# EUROPEAN PATENT APPLICATION

(11) **EP 3 907 279 A1**
(43) Date of publication of application: **10.11.2021**
(21) Application number: 19907557.3
(22) Date of filing: 17.01.2019
(51) Int. Cl.: C12N 5/079, A61K 35/30, A61P 25/00

(54) **NOVEL GLIA-LIKE CELLS DIFFERENTIATED FROM SOMATIC CELLS, PREPARATION METHOD THEREFOR, COCKTAIL COMPOSITION FOR PREPARING SAME, CELL THERAPEUTIC AGENT FOR PREVENTING OR TREATING NEUROLOGICAL DISORDERS, COMPRISING SAME, AND METHOD FOR PREVENTING AND TREATING NEUROLOGICAL DISORDERS BY ADMINISTERING SAME**

(30) Priority: 02.01.2019 KR 20190000465
(71) Applicant: Cellapeutics Bio, Bundang-gu Seongnam-si, Gyeonggi-do 13488 (KR)
(72) Inventor: KIM, Kyeong-Kyu, Seoul 06280 (KR); DE, Debojyoti, Suwon-si Gyeonggi-do 16421 (KR)
(74) Representative: EIP
(86) International application number: PCT/KR2019/000705
(87) International publication number: WO 2020/141648

(57) **Abstract**

The present disclosure relates to novel glia-like cells that are differentiated from somatic cells and secrete 20,000 pg/ml or more of HGF, a chemical cocktail composition for producing the same, a method for producing the same, a cell therapy product for treating neurological disorder containing the same, and a method of preventing and treating neurological disorder by administering the same.

## Description

### Technical Field

The present disclosure relates to novel glia-like cells that are differentiated from somatic cells, a method for producing the same, a cocktail composition for producing the same, a cell therapy product for treating neurological disorder containing the same, and a method of preventing and treating neurological disorder by administering the same.

### Background Art

Obtaining clinical grade cells has a number of significances in terms of disease modeling and drug efficacy testing, as well as the application of these cells in the treatment of several neurological disorders. It is possible to obtain physiologically active cells of different lineages from human iPSC and ES cells, but the broad application of these cells is hindered due to problems related to availability, immune susceptibility, functional integrity, oncogenic risk and efficiency issues (1-3). Therefore, transplantation of cells derived from a suitable immunotype-matched donor becomes an extremely important challenge.

Schwann cells (SCs) are major glial cells of the peripheral nervous system, and play an extremely important role in helping efficient nerve conduction by promoting nerve health while secreting several neurotrophic factors. In addition, SC cells play an essential role in the nerve regeneration process after peripheral nerve injury, and thus transplantation of SCs into the injured nerve area may be the only feasible therapeutic option for providing therapeutic benefits to patients with PNS and CNS injury by enhancing axonal regeneration.

Although Schwann cells may be the only therapeutic option for many neurological disorders, major difficulty in obtaining a sufficient amount of transplantable Schwann cells is because of the lack of autologous or allogeneic Schwann cells for this therapy. In many cases, healthy nerves from patients or allogeneic donors need to be excised as a source of transplantable Schwann cells. In addition, since cultured Schwann cells, which are final differentiated cells, have limited proliferation ability, the amount of cells sufficient to achieve any long-term therapeutic effect is limited.

In the case of iPSC cells, induced Schwann cells rely on efficient induction of neural crest and subsequent differentiation into Schwann cells having complex medium conditions and growth factors. On the other hand, differentiation of somatic cells into neural stem cells and functional neurons is achieved through forced expression of the master transcription factors *SOX10* and *Krox20.* Accordingly, Korean Patent Application Publication No. 10-2017-0045356 discloses a technology related to Schwann cells produced by introducing gene expression products. The introduction of these gene expression products may be effective in some aspects, but the wide use of these gene expression products in clinical practice has posed problems because of viral vector-mediated gene delivery, subsequent genetic manipulation, and the risk of side effects.

In addition, direct differentiation techniques using low-molecular compounds have also been developed, but in the case of cells that are differentiated by applying conventional low-molecular compounds, it has been pointed out that the development of cell therapy products is limited because the differentiated cells have the possibility of returning to their original cells when the low-molecular compounds are removed.

### Summary of Invention

The present disclosure relates to novel glia-like cells that are differentiated from somatic cells, a method for producing the same, a cocktail composition for producing the same, a cell therapy product for treating neurological disorder containing the same, and a method of preventing and treating neurological disorder by administrating the same.

### Technical Problem

The present disclosure is intended to solve the problems occurring in the prior art and provide novel glia-like cells (GLCs) that are capable of differentiating without genetic manipulation, may be obtained from somatic cells only by treatment with a chemical cocktail, and have not only an activity of regenerating and/or restoring injured nerves, but also an activity of protecting nerves themselves, like neuroglial cells derived from humans and/animals. As used herein, the term "neuroglial cells" includes oligodendroglias, astrocytes, Schwann cells, microglias, satellite cells, and ependymal cells.

### Technical Solution

The present disclosure provides glia-like cells that are differentiated from somatic cells and secrete 20,000 pg/ml or more of HGF.

The present disclosure also provides glia-like cells that are differentiated from somatic cells, wherein the glia-like cells are produced by a method including a differentiation induction step of inducing differentiation of the somatic cells by treatment with a first chemical cocktail containing a histone deacetylase inhibitor, a GSK inhibitor, an ALK-5 kinase inhibitor, a cAMP agonist and a histone demethylase inhibitor.

The present invention also provides a cocktail composition for producing glia-like cells containing a histone deacetylase inhibitor, a GSK inhibitor, an ALK-5 kinase inhibitor, a cAMP agonist and a histone demethylase inhibitor.

The present invention also provides a method of producing glia-like cells that are differentiated from somatic cells, the method including a differentiation induction step of inducing differentiation of the somatic cells by treatment with a first chemical cocktail containing a histone deacetylase inhibitor, a GSK inhibitor, an ALK-5 kinase inhibitor, a cAMP agonist and a histone demethylase inhibitor.

The present disclosure also provides a cell therapy product for treating neurological disorder containing, as an active ingredient, the above-described novel glia-like cells that are differentiated from somatic cells.

The present disclosure also provides a method of preventing and treating neurological disorder by administering the above-described glia-like cells that are differentiated from somatic cells.

### Advantageous Effects

As used herein, the term "neuroglial cells" refers to oligodendroglias, astrocytes, Schwann cells, microglias, satellite cells, and ependymal cells. The novel glia-like cells that are provided according to the present disclosure have the neuroregenerative, neurorestoring and/or neuroprotective function of neuroglial cells present *in vivo.*

In addition, the present disclosure induces direct differentiation of somatic cells having no differentiation ability, and thus aims to provide novel glia-like cells having no possibility of developing cancer, unlike pluripotent cells such as embryonic stem cells and dedifferentiated stem cells, which have been used in the development of cell therapy products in a conventional art.

In addition, the present disclosure uses only low-molecular compounds, and thus aims to provide a method for producing novel glia-like cells, which has less possibility of genome modification than a differentiation method composed of conventional genetic manipulations, shortens the differentiation period, has low differentiation costs, and may provide glia-like cells that are differentiated from somatic cells with excellent efficiency.

In addition, the present disclosure aims to provide a reculture technology capable of maintaining the differentiated cells as stable cells capable of being passaged in a common culture medium free of low-molecular compounds, thereby overcoming the instability of differentiated cells, which is one of the disadvantages of direct differentiation performed using conventional low-molecular compounds.

In addition, the present disclosure aims to provide a cell therapy product for preventing and/or treating neurological disorder containing novel nerve regeneration/protection functional cells that are differentiated from somatic cells, which are capable of repairing, regenerating and/or restoring injured nerve cells with excellent efficiency and have an activity of protecting nerve cells themselves.

In addition, the present invention aims to provide a cell therapy product, which may be obtained by direct differentiation of patient's somatic cells and used as a cell therapy product for neurological disorder, and thus has no immune rejection, unlike stem cell-based cell therapy products that have been previously used or studied.

According to the present disclosure, differentiation, dedifferentiation and/or cross-differentiation of somatic cells using a chemical cocktail has the advantages of easy application, including delivery, reproducibility and efficient scalability, and most importantly, may provide the effect of allowing an easy extrapolation for clinical purposes without any genetic manipulation.

In addition, the present disclosure has the effect of providing a method, which shortens the differentiation period compared to a differentiation method composed of conventional genetic manipulations and is capable of producing glia-like cells that are differentiated from somatic cells with excellent efficiency.

### Brief Description of Drawings

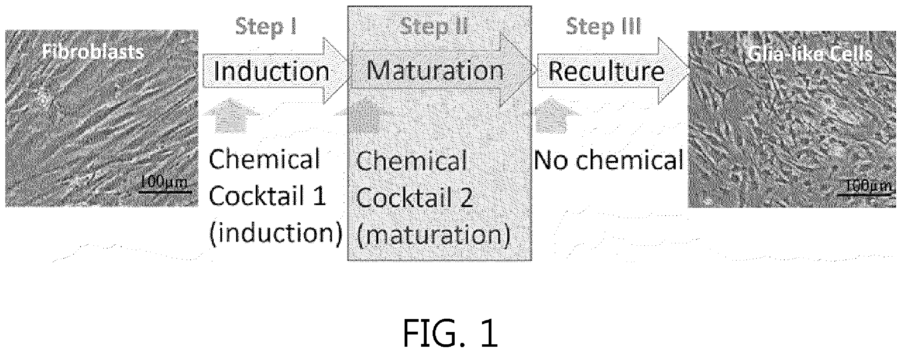

FIG. 1 shows an outline of a chemical-based conversion method in which fibroblasts are differentiated into novel glia-like cells (in the following examples and drawings corresponding thereto, abbreviated as "GLC"). Differentiation consists of three steps (step I : induction; step II : maturation; and step III: reculture). In the differentiation induction step which is step I, fibroblasts are converted into glia-like cells using a first chemical cocktail, and in the maturation step which is step II, the converted glia-like cells are matured using a second chemical cocktail. In the reculture step which is step III, stable cells are cultured in a culture medium free of the compounds (chemicals) used in the differentiation. At this time, the second maturation step is not necessarily required.
FIG. 2 illustrates compounds used for differentiation into glia-like cells.
FIG. 3 is a microscopic photograph showing the morphological characteristics of novel glia-like cells that are differentiated from somatic cells (FIG. 3 shows the morphological characteristics of novel glia-like cells obtained by inducing differentiation using a first chemical cocktail containing the six compounds (VCRFPT) shown in FIG. 2 and then maturing the cells using a second chemical cocktail containing three compounds (CRF); scale bar=100 µm).
FIG. 4 shows the morphological characteristics of novel glia-like cells obtained by inducing differentiation using a chemical cocktail composed of only VCRFT among the components of the first chemical cocktail (scale bar=100 µm), and indicates that the efficiency of differentiation was low.
FIG. 5 shows the morphological characteristics of novel glia-like cells obtained by inducing differentiation using a chemical cocktail composed of only CRFPT among the components of the first chemical cocktail (scale bar=100 µm), and indicates that the efficiency of differentiation was low.
FIG. 6 shows the morphological characteristics of novel glia-like cells obtained by inducing differentiation using a chemical cocktail composed of only VRFPT among the components of the first chemical cocktail (scale bar=100 µm), and indicates that the efficiency of differentiation was low.
FIG. 7 shows the morphological characteristics of novel glia-like cells obtained by inducing differentiation using a chemical cocktail composed of only VCFPT among the components of the first chemical cocktail (scale bar=100 µm), and indicates that the efficiency of differentiation was low.
FIG. 8 shows the morphological characteristics of novel glia-like cells obtained by inducing differentiation using a chemical cocktail composed of only VCRPT among the components of the first chemical cocktail (scale bar=100 µm), and indicates that no differentiation was induced in the absence of "F" (Forskolin), suggesting that "F" is essential for differentiation.
FIG. 9 shows the morphological characteristics of novel glia-like cells obtained by inducing differentiation using a chemical cocktail composed of only VCRFT among the components of the first chemical cocktail (scale bar=100 µm), and indicates that the efficiency of differentiation was low.
FIG. 10 is a microscopic photograph showing the morphological characteristics of novel glia-like cells that are differentiated from somatic cells, and indicates that the efficiency of differentiation of the somatic cells into the glia-like cells was low when a chemical cocktail consisting of VCRF was used.
FIG. 11 is a microscopic photograph showing the morphological characteristics of novel glia-like cells that are differentiated from somatic cells, and indicates that differentiation of the somatic cells into the glia-like cells was well induced by a first chemical cocktail consisting of VCRFP. Thus, it was confirmed that differentiation of fibroblasts into glia-like cells could occur even in the absence of "T" (scale bar=100 µm).
FIG. 12 illustrates other kinds of compounds used for differentiation into glia-like cells. The compounds used have the same functions as those of the compounds shown in FIG. 2.
FIG. 13 is a microscopic photograph showing the morphological characteristics of novel glia-like cells that are differentiated from somatic cells. FIG. 13 shows the morphological characteristics of novel glia-like cells obtained by inducing differentiation from fibroblasts using a first chemical cocktails consisting of a combination of the compounds shown in FIG. 6 (scale bar=100 µm).
FIG. 14 shows a protocol for a chemical-based conversion method for allowing human fibroblasts to differentiate into glia-like cells, in which the period of the maturation step varies in a state in which the period of the differentiation induction step is fixed to 3 days.
FIG. 15 shows a protocol for a chemical-based conversion method for allowing human fibroblasts to differentiate into glia-like cells, in which the differentiation induction step is performed during varying periods (days), followed by the maturation step.
FIG. 16 shows a protocol for a chemical-based conversion method for allowing human fibroblasts to differentiate into glia-like cells, which includes a step of re-culturing the cells without treatment with any chemical cocktail.
FIG. 17 is a micrograph showing the morphology of human Schwann cells.
FIG. 18 is a photograph of glia-like cells (C1-GLC) produced according to protocol 1 by treatment with a combination of VCRFPT as a first cocktail compound and CRF as a second chemical cocktail, and indicates that the glia-like cells have a morphology very similar to that of human Schwann cells (FIG. 17), but have a morphology different from that of fibroblasts (FIG. 21).
FIG. 19 is a photograph of glia-like cells (C2-GLC) produced according to protocol 2 by treatment with a combination of VCRFPT as a first cocktail compound and CRF as a second chemical cocktail, and indicates that the glia-like cells have a morphology similar to that of human Schwann cells (FIG. 17), but have a morphology different from that of fibroblasts (FIG. 21).
FIG. 20 is a photograph of glia-like cells (C3-GLC) produced according to protocol 3 by treatment with a combination of VCRFPT as a first cocktail compound and CRF as a second chemical cocktail, and indicates that the glia-like cells have a morphology similar to that of human Schwann cells (FIG. 17), but have a morphology different from that of fibroblasts (FIG. 21).
FIG. 21 is a micrograph of fibroblasts that are somatic cells.
FIG. 22 depicts microscopic photographs. FIG. 22A shows the morphological characteristics of human fibroblasts used for differentiation, FIG. 22B shows the morphological characteristics of glia-like glia (Class 3 Glia-like cells, C3-GLC) obtained by differentiation, and FIG. 22C shows the morphological characteristics of human Schwann cells. In order to show the morphological characteristics of each type of cells, the outside of several cells showing a representative morphology is marked with a red dotted line (scale bai=100 µm).
FIG. 23 shows unsupervised clustering of Schwann cell marker genes differentially expressed in each sample as analyzed by microarray (N_Hum_Schw: human Schwann cell; N_fibroblast: fibroblast; N_SC_12d: Cl-GLC(6+3); N_SC_9d: Cl_GLC(6+6)). It can be confirmed that Schwann cell markers are expressed in differentiated cells and Schwann cells, but Schwann cell markers are hardly expressed in fibroblasts. Drawings related to more specific expressed substances are described below (FIGS. 71 to 74).
FIG. 24 shows unsupervised clustering of oligodendrocyte marker genes differentially expressed in each sample as analyzed by microarray (N_Hum_Schw: human Schwann cell; N_fibroblast: fibroblast; N_SC_12d: Cl-GLC(6+3); N_SC_9d: Cl_GLC(6+6)). It can be confirmed that many genes that are expressed in oligodendrocytes are expressed in differentiated cells, but oligodendrocyte markers are not expressed in fibroblasts and some of oligodendrocyte markers are expressed in Schwann cells.
FIG. 25 shows unsupervised clustering of astrocyte marker genes differentially expressed in each sample as analyzed by microarray (N_Hum_Schw: human Schwann cell; N_fibroblast: fibroblast; N_SC_12d: Cl-GLC(6+3); N_SC_9d: Cl_GLC(6+6)). It can be confirmed that many genes that are expressed in astrocytes are expressed in differentiated cells, but astrocyte marker genes are not expressed in fibroblasts and some of astrocyte marker genes are expressed in Schwann cells.
FIG. 26 shows the results of functionally classifying genes whose expression in Cl-GLC(6+6) increases as analyzed by microarray, and indicates that the expression of cytokines that are expressed in neuroglial cells such as astrocytes and microglias increases.
FIG. 27 shows the results of microarray analysis of marker genes expressed in glia-like cells (GLCs), fibroblasts and human Schwann cells, which were differentiation-induced and matured during different periods (days). (A) expression of neuroglial marker genes, and (B) expression of fibroblast marker genes.
FIG. 28 shows C1-GLC stained by immunofluorescence. A, B and C are photographs obtained using S100, P0 (MPZ) and GFAP as neuroglia markers, respectively. DAPI was commonly used to stain the nucleus (scale bar=100 µm).
FIG. 29 shows C3-GLC stained by immunofluorescence using GFAP (green) as a neuroglia marker. DAPI (blue) was used to stain the nucleus, and EDU (red) was used to stain proliferating cells (scale bar=50 µm).
FIG. 30 depicts specific graphs showing the results of measuring the expression levels of fibroblast marker genes in glia-like cells obtained by differentiation according to protocol I, somatic cells and fibroblasts. The expressed levels were measured by RT-PCR and compared.
FIG. 31 depicts specific graphs showing the results of measuring the expression levels of fibroblast marker genes in glia-like cells obtained by differentiation according to protocol II, somatic cells and fibroblasts. The expressed levels were measured by RT-PCR and compared.
FIG. 32 depicts specific graphs showing the results of measuring the expression levels of neuroglia marker genes in glia-like cells obtained by differentiation according to protocols II and III, somatic cells and fibroblasts. The expressed levels were measured by RT-PCR and compared.
FIG. 33 shows the results of analyzing the amounts of cytokines and growth factors secreted into culture media of glia-like cells. It can be confirmed that the amounts of MIF, CXCL12, IL8, BDNF, GRO-alpha, HGF and the like secreted from glia-like cells are larger than the amounts of those from fibroblasts. It can be confirmed that MLF, BDNF, HGF and the like, known to have nerve protection and regeneration functions, are more highly expressed in glia-like cells than human Schwann cells (hSC).
FIGS. 34 to 39 are graphs showing the results of quantitatively analyzing cytokines and growth factors secreted from different glia-like cells produced according to protocols I, II and III, respectively. The amounts of proteins secreted into media during 3 days of culture of 3.2 × 10⁶ cells in a 60-mm culture dish having an area of 28.2 cm² were measured by ELISA.
FIG. 40A is a representative micrograph of neurite outgrowth of motor neuron NSC34 cells treated with a somatic cell (fibroblast) culture medium, and FIG. 40B is a graph showing the results of quantitatively analyzing the neurite length of motor neuron NSC34 cells treated with a fibroblast culture medium.
FIG. 41A is a representative micrograph of neurite outgrowth of motor neuron NSC34 cells treated with a culture medium of Cl-GLC(6+6) glia-like cells obtained by differentiation according to protocol I, and FIG. 41B is a graph showing the results of quantitatively analyzing the neurite length of motor neuron NSC34 cells treated with a culture medium of Cl-GLC(6+6) glia-like cells obtained by differentiation according to protocol I.
FIG. 42A is a representative micrograph of neurite outgrowth of motor neuron NSC34 cells treated with a culture medium of C2-GLC(15+3) glia-like cells obtained by differentiation according to protocol II, and FIG. 42B is a graph showing the results of quantitatively analyzing the neurite length of motor neuron NSC34 cells treated with a culture medium of C2-GLC(15+3) glia-like cells obtained by differentiation according to protocol II.
FIG. 43A is a representative micrograph of neurite outgrowth of motor neuron NSC34 cells treated with a culture medium of C3-GLC(6+3+12) glia-like cells obtained by differentiation according to protocol III, and FIG. 43B is a graph showing the results of quantitatively analyzing the neurite length of motor neuron NSC34 cells treated with a culture medium of C3-GLC(6+3+12) glia-like cells obtained by differentiation according to protocol III.
FIG. 44A is a representative micrograph of neurite outgrowth of motor neuron NSC34 cells treated with a Schwann cell culture medium, and FIG. 44B is a graph showing the results of quantitatively analyzing the neurite length of motor neuron NSC34 cells treated with a NSC34 Schwann cell culture medium.
FIG. 45 shows the rate of conversion into glia-like cells, which corresponds to a yield of 85% as measured by cells exhibiting GFAP expression.
FIGS. 46A-46C show the proliferation characteristics of recultured cells. FIG. 46A shows the degree of EdU staining (red) of C3-GLC(6+3+12) glia-like cells obtained by differentiation according to protocol III, which is an estimate of cell proliferation ability, and
FIG. 46B shows a nucleus stained with Hoechst33342 (blue), and FIG. C is a merged image of FIGS. 46A and 46B (scale bar=50 µm).
FIG. 47 is a quantitative graph showing the percentage of EdU-positive cells. The error bar means the standard error of mean (SEM).
FIG. 48 is a microscopic photograph showing the morphological characteristics of C3-GLC(6+3+6) glia-like cells thawed after freezing. It shows that there is no change in cell morphology even after freezing and thawing (scale bar=50 µm).
FIG. 49 is a quantitative analysis graph showing the results of ELISA performed to measure the amounts of HGF secreted from different glia-like cells produced according to protocols I, II and III. In particular, in the case of C3-GLC (6+3+6) and C3-GLC (6+3+12), the amount of HGF secreted during cell growth after cell freezing (F) and thawing (T) (FT) was measured. FIG. 49 shows that there is no significant change in HGF expression even after freezing and thawing.
FIG. 50 is a photograph showing neurite outgrowth of NSC34 cells in a culture medium of C3-GLC (6+3+12) glia-like cells obtained according to protocol III, and shows that there is no change in cell morphology and neurite outgrowth even after freezing and thawing (scale bar=100 µm).
FIG. 51 is a graph showing the results of quantitatively analyzing neurite outgrowth of NSC34 cells in a culture medium of C3-GLC (6+3+12) glia-like cells obtained according to protocol III, and shows that there is no change in cell morphology and neurite outgrowth even after freezing and thawing.

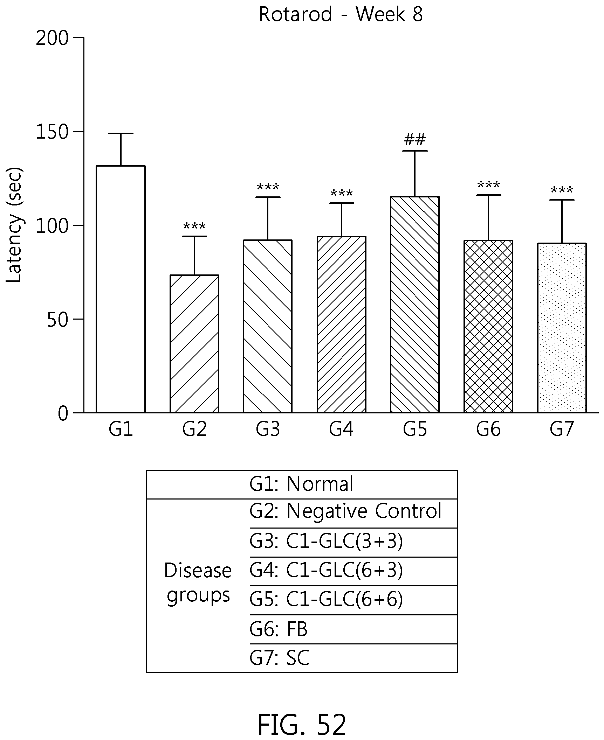

FIG. 52 is a graph showing the results of rotarod analysis. A rat chronic constriction injury (CCI) model was created by injuring rat femoral nerves, and at 8 weeks after transplanting human fibroblasts, human Schwann cells or glia-like cells into the injured nerves, the degree of nerve regeneration was measured by rotarod analysis. It can be confirmed that nerve regeneration was better in FB (fibroblasts), SC (Schwann cells), G3, G4 and G5 (C1-GLC) than in G2 (untreated), G6 (treated with fibroblasts) and G7 (treated with human Schwann cells). In particular, it can be confirmed that nerve regeneration was best in G5 (C1-GL 6+6).
FIG. 53 is a graph showing the results of EMG analysis. An experiment was conducted under the same conditions as in FIG. 37, and the degree of nerve regeneration was analyzed by electromyogram (EMG). It can also be confirmed that nerve regeneration was better in G5 (treated with C1-GLC (6+6)) than in G2 (untreated), G6 (treated with fibroblasts) and G7 (treated with human Schwann cells).
FIG. 54 depicts photographs showing myelinated nerves. An experiment was conducted under the same conditions as in FIG. 52, and the degree of nerve regeneration was analyzed based on the distribution of myelinated neurons. It can be confirmed that myelination was better in G5 (treated with C1-GLC (6+6)) than in G2 (untreated), G6 (treated with fibroblasts) and G7 (treated with human Schwann cells).
FIG. 55 is a quantitative graph showing the myelinated neurons in the photographs of FIG. 54. It can be confirmed that the number of myelinated neurons was larger in G5 (treated with C1-GLC (6+6)) than in G2 (untreated), G6 (treated with fibroblasts) and G7 (treated with human Schwann cells), indicating that nerve regeneration was best in G5.
FIG. 56 depicts electron micrographs of myelinated neurons. An experiment was conducted under the same conditions as in FIG. 52, and the degree of nerve regeneration was analyzed based on the degree of myelination of neurons. It can be confirmed that myelination was better in G5 (treated with C1-GLC (6+6)) than in G2 (untreated), G6 (treated with fibroblasts) and G7 (treated with human Schwann cells).
FIG. 57 is a graph showing the results of quantifying the myelin thickness based on the degree of myelination of neurons in the electron micrographs of FIG. 56. It can be confirmed that the myelin thickness was larger in G5 (treated with C1-GLC (6+6)) than in G2 (untreated), G6 (treated with fibroblasts) and G7 (treated with human Schwann cells), indicating that nerve regeneration was best in G5.
FIG. 58 shows the concentrations of six kinds of compounds contained in a chemical cocktail used in a process of producing glia-like cells by inducing differentiation. FIG. 58 shows the working concentration, 2x working concentration, IC₅₀ values and 2x IC₅₀ values of each compound, and FIGS. 59 to 62 show microscopic photographs of glia-like cells obtained when inducing differentiation using cocktails containing different concentrations of compounds (scale bar=100 µm).
FIG. 59 is a photograph showing glia-like cells obtained by inducing differentiation by treatment with a cocktail containing working concentrations of compounds. It could be confirmed that differentiation was well induced in view of the morphology of the glia-like cells.
FIG. 60 is a photograph showing glia-like cells obtained by inducing differentiation by treatment with a cocktail containing 2x working concentrations of compounds. It was confirmed that, although differentiation was induced, the number of converted cells decreased, indicating that the compounds were somewhat toxic to the cells.
FIG. 61 is a photograph showing glia-like cells obtained by inducing differentiation by treatment with a first chemical cocktail containing six compounds at concentrations corresponding to IC₅₀ values, and indicates that the efficiency of differentiation significantly decreased.
FIG. 62 is a photograph showing glia-like cells obtained by inducing differentiation by treatment with a chemical cocktail containing six compounds at concentrations corresponding to 2x IC₅₀ values, and indicates that the efficiency of differentiation significantly decreased.
FIGS. 63 and 64 relate to glia-like cell differentiation from fibroblasts isolated from patient's skin cells, and are micrographs of C2-GLC (15+3) and C3-GLC( 6+3+6) glia-like cells converted using protocols II (FIG. 63) and III (FIG. 64) from fibroblasts collected from the skin of a patient suffering from CMK (Charcot-Marie-Tooth) disease (scale bai=100 µm).
FIGS. 65 and 66 relate to glia-like cell differentiation from dermis-derived fibroblasts of a 6-year-old male, and are micrographs of C2-GLC (15+3) and C3-GLC( 6+3+6) glia-like cells obtained by converting the fibroblasts using protocols II (FIG. 65) and III (FIG. 66) (scale bar=100 µm).
FIGS. 67 and 68 relate to glia-like cell differentiation from skin-derived fibroblasts of a 82-year-old female, and are micrographs of C2-GLC (15+3) and C3-GLC( 6+3+6) glia-like cells obtained by converting the fibroblasts using protocols II (FIG. 67) and III (FIG. 68) (scale bar=100 µm).
FIGS. 69 and 70 relate to glia-like cell differentiation from foreskin-derived fibroblasts of a 47-year-old male, and are micrographs of C2-GLC (15+3) and C3-GLC( 6+3+6) glia-like cells obtained by converting the fibroblasts using protocols II (FIG. 69) and III (FIG. 70) (scale bar=100 µm).
FIGS. 71 to 74 specifically show unsupervised clustering of Schwann cell marker genes differentially expressed in each sample as identified by microarray in FIG. 23 (N_Hum_Schw: human Schwann cells; N_fibroblast: fibroblasts; N_SC_12d: Cl-GLC(6+3); N_SC_9d: C1-GLCC6+6)).
FIG. 75 is a graph showing the amount of HGF secreted from glia-like cells (C3-GLC 6+3+6) (FIG. 64) produced from CMT patient's fibroblasts.
FIG. 76 is a graph showing the amount of HGF secreted from glia-like cells (C2-GLC(15+3) obtained by differentiation from various fibroblasts according to the method of protocol II.
FIG. 77 is a graph showing the amount of HGF secreted from glia-like cells (C2-GLC(6+6) produced according to the method of protocol I using various combinations of the components of the chemical cocktail used in the differentiation induction step.
FIG. 78 is a graph showing the amount of HGF secreted from glia-like cells produced according to the methods of protocols II and III.
FIG. 79 is a graph showing the amounts of HGF secreted from fibroblasts, Schwann cells, and glia-like cells produced by differentiation using a combination of chemical cocktail compounds associated with the concentrations of chemical cocktail compounds shown in FIG. 58.

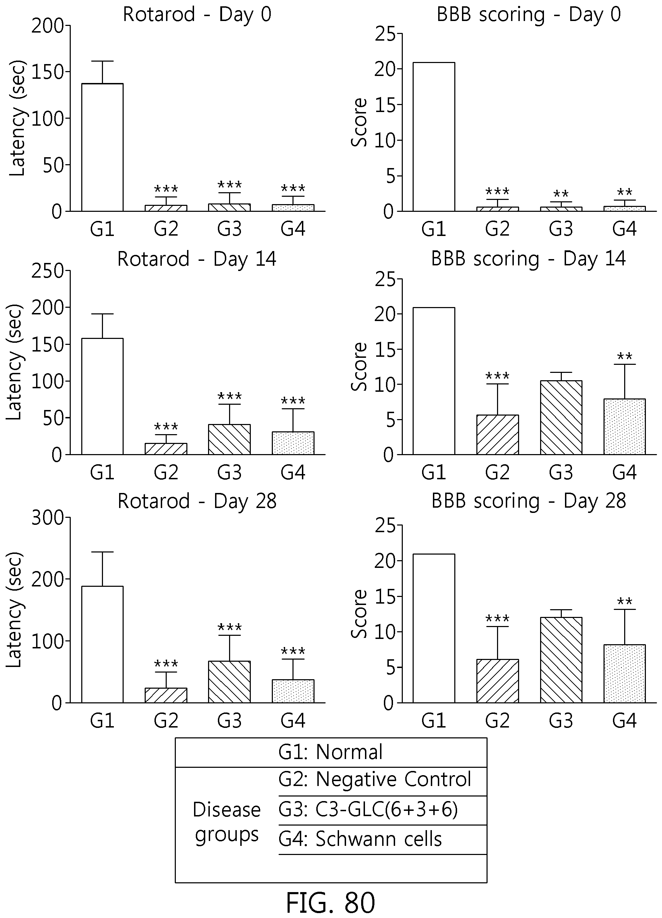

FIG. 80 depicts the results of rotarod analysis and BBB scoring. A spinal cord injury (SCI) rat model was created by injuring rat spinal nerves, and on 0, 14 and 28 days after transplanting human Schwann cells and glia-like cells into the injured nerves, the degree of nerve regeneration was measured by rotarod analysis and BBB scoring (BBB scoring: Basso, Beattie and Breshen locomotor scale method). It can be confirmed that nerve regeneration was better in FB (fibroblasts), SC (Schwann cells) and G3 (treated with C3-GLC (6+3+6)) than in G2 (untreated) and G4 (treated with human Schwann cells).
FIG. 81 shows the *in vivo* distribution of glia-like cells. C3-GLC and human Schwann cells showing green fluorescence by infection with a virus having a green fluorescence protein gene were injected into the thigh muscles of rats using a syringe, and then the location and brightness of the fluorescence were observed at a predetermined time point, and change in C3-G(6+3+6) was observed. As a result of the experiment, it could be confirmed that the glia-like cells remained for at least 5 days.

### Detailed Description of Embodiments of Invention

Hereinafter, the present disclosure will be described in detail.

The present disclosure is intended to solve the problems occurring in the prior art and provide novel glia-like cells (GLCs) that are capable of differentiating without genetic manipulation, may be obtained from somatic cells only by treatment with a chemical cocktail, and have not only an activity of regenerating and/or restoring injured nerves, but also an activity of protecting nerves themselves, like neuroglial cells derived from humans and/animals.

As used herein, the term "neuroglial cells" includes oligodendroglias, astrocytes, Schwann cells, microglias, satellite cells, and ependymal cells. The novel glia-like cells that are provided according to the present disclosure have the neuroregenerative, neurorepairing and/or neuroprotective function of neuroglial cells.

In addition, the present disclosure induces direct differentiation of somatic cells having no differentiation ability, and thus aims to provide novel glia-like cells having no possibility of developing cancer, unlike pluripotent cells such as embryonic stem cells and dedifferentiated stem cells, which have been used in the development of cell therapy products in a conventional art.

In addition, the present disclosure uses only low-molecular compounds, and thus aims to provide a method for producing novel glia-like cells, which has less possibility of genome modification than a differentiation method composed of conventional genetic manipulations, shortens the differentiation period, and may provide glia-like cells that are differentiated from somatic cells with excellent efficiency.

In addition, the present disclosure aims to provide a reculture technology capable of maintaining the differentiated cells as stable cells capable of being passaged in a common culture medium free of low-molecular compounds, thereby overcoming the instability of differentiated cells, which is one of the disadvantages of direct differentiation performed using conventional low-molecular compounds. As used herein, the term "low-molecular compounds" refers to components that may constitute the first chemical cocktail or second chemical cocktail that is included in the present disclosure.

In addition, the present disclosure aims to provide a cell therapy product for preventing or treating neurological disorder containing novel neuroregenerative/neuroprotective cells that are differentiated from somatic cells, which are capable of repairing, regenerating and/or restoring injured nerve cells with excellent efficiency and have an activity of protecting nerve cells themselves.

In addition, the present invention aims to provide a cell therapy product, which may be obtained by direct differentiation of patient's somatic cells and used as a cell therapy product for neurological disorder, and thus had no immune rejection, unlike stem cell-based cell therapy products that have been previously used or studied.

The novel glia-like cells of the present disclosure may secrete 20,000 pg/ml or more of HGF.

The present disclosure also provides novel glia-like cells that are differentiated from somatic cells and secrete 10 ng/ml or more of MIF and 150 pg/ml or more of BNDF. The glia-like cells may be for nerve regeneration and protection, and specifically, make it possible to regenerate and restore injured nerve cells with excellent efficient and/or protect nerves.

More specifically, the amount of HGF (which is a protein factor having a neuroregenerative function) secreted from the novel glia-like cells of the present disclosure may be at least 10 times higher than the amount secreted from fibroblasts used for differentiation, or may be at least 20,000 pg/ml, preferably 20,000 to 5,000,000 pg/ml, more preferably 30,000 to 5,000,000 pg/ml, even more preferably 50,000 to 5,000,000 pg/ml. Since HGF is a protein that has neuroprotective and neuroregenerative functions, it may exhibit a better effect as the secretion thereof increases. In addition, the amount of BDNF (which is another protein factor having a neuroregenerative function) secreted from the novel glia-like cells of the present disclosure may be at least 10 times higher than the amount secreted from fibroblasts used for differentiation, or may be at least 150 pg/ml, preferably 200 to 5,000,000 pg/ml, more preferably 700 to 5,000,000 pg/ml, even more preferably 1,000 to 5,000,000 pg/ml. Since BDNF is also a protein that has neuroprotective and neuroregenerative functions, it may exhibit a better effect as the secretion thereof increases. In addition, the amount of MIF (which is another protein factor having a neuroregenerative function) secreted from the novel glia-like cells of the present disclosure may be at least 10 times higher than the amount secreted from fibroblasts used for differentiation, or may be at least 10 ng/ml, preferably 15 to 1,000,000 ng/ml, more preferably 20 to 1,000,000 ng/ml. Since MIF is a protein having neuroprotective and neuroregenerative functions, it may exhibit a better effect as the secretion thereof increases. Since HGF, MIF and BDNF are protein factors known to have neuroprotective and neuroregenerative functions, they may have excellent effects in terms of neuroregenerative function when secreted in amounts within the above-described ranges.

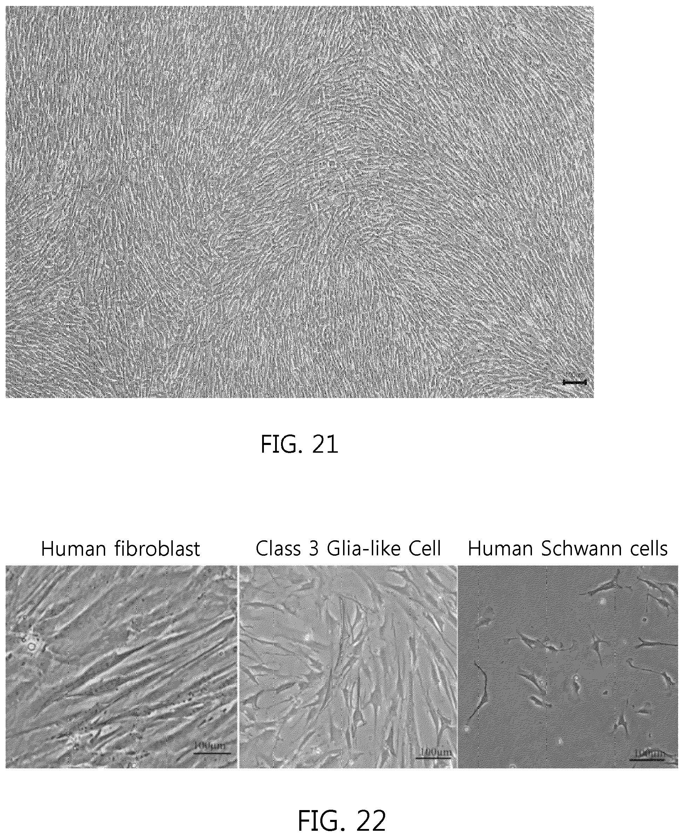

In addition, the glia-like cells show a morphology similar to that of human Schwann cells. More specifically, the glia-like cells are elongate cells having a length of 50 to 500 vL and a diameter of 15 to 50 vL, and taper from the bulging center to both ends or several ends (<10), and in some cases, this end portion has a long thread shape of about 1 micron in width (see FIG. 22).

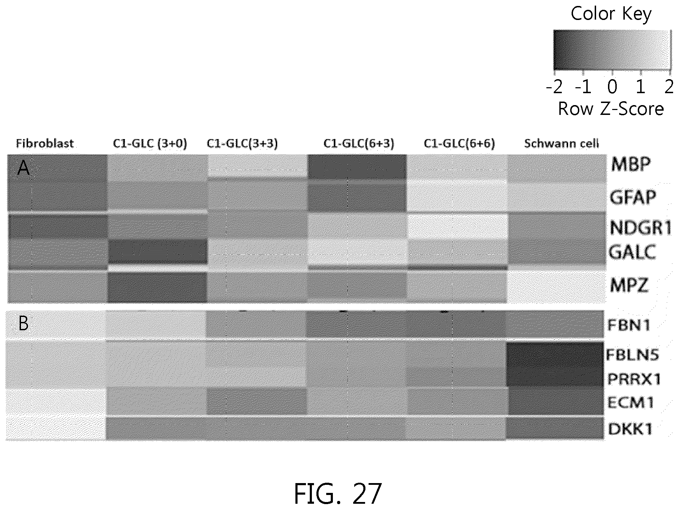

In addition, the glia-like cells are characterized in that the expression of the marker DKK1 or FBLN5 (of fibroblasts used for differentiation) therein is observed to be 20% or less of the expression thereof in fibroblasts, and the expression of MBP, GALC, NDRG1 and the like, which are marker genes known to have increased expression in neuroglial cells, increases at least 1.5 times compared to that in fibroblasts (see FIG. 27).

In addition, the glia-like cells are characterized by enhancing neurite outgrowth of motor neurons, like NSC-32. More specifically, when the glia-like cell culture medium is added during culture of motor neurons, 70% or more of the cells have neurites having a length of 50 to 500 µm.

The present disclosure also provides a first chemical cocktail and a second chemical cocktail, which make it possible to produce the novel glia-like cells.

The first chemical cocktail serves to induce somatic cell differentiation into the novel glia-like cells, and may contain a histone deacetylase inhibitor, a GSK inhibitor, an ALK-5 kinase inhibitor, a cAMP agonist and a histone demethylase inhibitor.

In addition, the second chemical cocktail serves to post-treat the somatic cells treated with the first chemical cocktail in order to mature the somatic cells, and may contain a GSK inhibitor, an ALK-5 kinase inhibitor and a cAMP agonist.

In the present disclosure, the term "histone deacetylase inhibitor" refers to a substance that inhibits histone deacetylase. It is known that the histone deacetylase inhibitor exhibits a strong cytostatic anticancer activity by promoting the expression of cell proliferation inhibitory factors and genes necessary for inducing differentiation by forming chromatin in a highly acetylated state to induce the differentiation of cancer cells and inhibit angiogenesis, and causing apoptosis of the cancer cells by arresting the cell cycle in the G1 state. The histone deacetylase (HDAC) inhibits gene transcription via pRB/E2F, and the breakdown in histone acetylation is involved in the generation of various types of cancer. The HDAC is highly expressed under severe environmental conditions such as hypoxia, hypoglycemia, cell carcinogenesis, etc. to promote cell proliferation by inhibiting the expression of cell proliferation inhibitory factors, and is known to be recognized as a key regulatory factor for cell carcinogenesis and differentiation regulation. Particularly, it is known that the VPA induces inositol reduction, inhibits GSK-3β, activates an ERK pathway, and stimulates PPAR activation. The histone deacetylase (HDAC) inhibitor may be contained at a concentration of 1 nM to 100 mM

The histone deacetylase inhibitor is not limited in the kind thereof, but more specifically, may include at least one selected from the group consisting of valproic acid, pracinostat, trichostatin A, 2,4-pyridinedicarboxylic acid, suberoylanilide hydroxamic acid, hydroxamic acid, cyclic tetrapeptide, depsipeptides, vorinostat, belinostat, panobinostat, benzamide, entinostat, and butyrate. More preferably, the histone deacetylase inhibitor may be valproic acid and/or pracinostat.

Among the above-described compounds, valproic acid may be contained at a concentration of 50 µM to 5 mM, preferably 250 µM to 4 mM, more preferably 400 µM to 2 mM, even more preferably 500 µM to 1 mM. When valproic acid is contained at a concentration within the above range, it may be preferable in terms of being effective and able of exhibiting an effect without toxicity. In addition, when pracinostat among the above compounds is contained at a concentration of 50 nM to 200 nM, it may be preferable in terms of being able to differentiate somatic cells into glia-like cells in an excellent manner without toxicity.

In the present disclosure, the "GSK (glycogen synthase kinase) inhibitor" is not limited as long as it is a substance that targets GSK1/2 which is an upstream molecule involved in the GSK signaling pathway. The GSK (glycogen synthase kinase) inhibitor may be contained at a concentration of 1 nM to 100 mM. The GSK inhibitor is not limited in the kind thereof, but more specifically, may be at least one selected from the group consisting of Chir99021 (6-(2-(4-(2,4-dichlorophenyl)-5-(4-methyl-1H-imidazol-2-yl)pyrimidin-2-ylamino)ethylamino)nicotinonitrile); LY2090314 (3-imidazo[1,2-a]pyridin-3-y1-4-[1,2,3,4-tetrahydro-2-(1-piperidinylcarbonyl)-pyrrolo[3,2,jk][1,4] benzodiazepin-7-yl]); 1-azakenpaullone(9-bromo-7,12-dihydro-pyrido[3',2:2,3]azepino[4,5-b]indol-6(5H)-one); BIO ((2'Z,3'E)-6-bromoindirubin-3'-oxime); ARA014418 (N-(4-methoxybenzy1)-N'-(5-nitro-1,3-thiazol-2-yl)urea); indirubin-3'-monoxime; 5-iodo-indirubin-3'-monoxime; kenpaullone (9-bromo-7,12-dihydroindolo-[3,2-d] [1]benzazepin-6(5H)-one); SB-415286 (3-[(3-chloro4-hydroxyphenyl)amino]-4-(2-nitro-phenyl)-1H-pyrrole-2,5-dione); SB-216763 (3-(2,4-dichloropheny1)4-(1-methy1-1H- indol-3-yl)-1H-pyrrole-2,5-dione); Maybridge SEW00923SC (2-anilino-5-phenyl-1,3,4-oxadiazole); (Z)-5-(2,3-methylenedioxyphenyl)-imidazolidine-2,4-dione; TWS 119 (3-(6-(3-aminophenyl)-7H-pyrrolo[2,3-d]pyrimidin-4-yloxy)phenol); Chir98014 (N2-(2-(4-(2,4-dichlorophenyl)-5-(1H-imidazol-1-yl)pyrimidin-2-ylamino)ethyl)-5-nitropyridine-2,6-diamine); SB415286 (3-(3-chloro-4-hydroxyphenylamino)-4-(2-nitrophenyl)-1H-pyrrole-2,5-dione); and Tideglusib (2-(1-naphthalenyl)-4-(phenylmethyl)). Preferably, the GSK inhibitor may be Chir 99021 and/or LY2090314.

Among the GSK inhibitors, Chir 99021 and/or LY2090314 may be contained at a concentration of 5 nM to 1 mM, preferably 10 nM to 20 µM, more preferably 20 nM to 15 µM, even more preferably 10 µM to 20 µM. When Chir 99021 and/or LY2090314 are/is contained at a concentration within the above range, it may be preferable in terms of being effective and being able to exhibit an effect without toxicity.

In the present disclosure, the term "ALK-5 kinase inhibitor" refers to a substance that interferes with normal signaling of TGF-β type I by binding to TGF-β type I receptor. TGF-β type I (transforming growth factor-β type I) is a multifunctional peptide having various effects on cell proliferation, differentiation and various types of cells. This multifunctionality is known to play a pivotal role in the growth and differentiation of various tissues, including adipocyte formation, myocyte formation, bone cell formation, and epithelial cell differentiation.

The ALK-5 kinase inhibitor may be contained at a concentration of 1 nM to 100 mM

The ALK-5 kinase inhibitor is not limited in the kind thereof. More specifically, the ALK-5 kinase inhibitor (TGF-β type I receptor inhibitor) may be at least one selected from the group consisting of RepSox (1,5-naphthyridine, 2-[3-(6-methyl-2-pyridinyl)-1H-pyrazol-4-yl]); SM31542 (4-(4-(benzo[d][1,3]dioxol-5-yl)-5-(pyridin-2-yl)-1H-imidazol-2-yl)benzamide); SB525334 (6-(2-tert-butyl-(6-methylpyridin-2-yl)-1H-imidazol-5-yl)quinoxaline); GW788388 (4-(4-(3-(pyridin-2-yl)-1H-pyrazol-4-yl)pyridin-2-yl)-N-(tetrahydro-2H-pyran-4-yl)benzamide); SD-208 (2-(5-chloro-2-fluorophenyl)-N-(pyridin-4-yl)pteridin-4-amine); Galunisertib (LY2157299, 4-(2-(6-methylpyridin-2-yl)-5,6-dihydro-4H-pyrrolo[1,2-b]pyrazol-3-yl)quinolone-6-carboxamide); EW-7197 (N-(2-fluorophenyl)-5-(6-methy1-2-pyridinyl)-4-[1,2,4]triazolo[1,5-a]pyridin-6-yl-1H-imidazole-2-methanamine); LY2109761 (7-(2-morpholinoethoxy)-4-(2-(pyridin-2-yl)-5,6-dihydro-4H-pyrrolo[1,2-b]pyrazol-3-yl)quinoline); SB505124 (2-(4-(benzo[d][1,3]dioxol-5-yl)-2-tert-butyl-1H-imidazol-5-yl)-6-methylpyridine); LY364947 (quinoline, 4-[3-(2-pyridinyl)-1H-pyrazo1-4-y1 ]); K02288 (3-1(6-amino-5-(3,4,5-trimethoxyphenyl)-3-pyridinyl]phenol]; and LDN- 212854 (quinoline, 5-[6-[4-(1-piperazinyl)phenyl]pyrazolo[1,5-a]pyrimidin-3-yl]). Preferably, the ALK-5 kinase inhibitor may be Repsox and/or SB431542.

More specifically, Repsox and/or SB431542 may be contained at a concentration of 1 nM to 500 µM, preferably 4 nM to 20 µM, more preferably 8 nM to 7.5 µM, even more preferably 5 µM to 10 µM. When Repsox and/or SB431542 are/is contained at a concentration within the above range, it may be preferable in terms of being effective and being able to exhibit an effect without toxicity.

In the present disclosure, the term "cAMP agonist" refers to a substance that activates cAMP signaling. The cAMP agonist may be contained at a concentration of 1 nM to 100 mM

The cAMP agonist is not limited in the kind thereof, but specifically, may include forskolin, isoproterenol, NKH 477 (a novel forskolin derivative), PACAP 1-27 (pituitary adenylate cyclase activating polypeptide receptor antagonist; PACAP antagonist), or PACAP 1-38 (PACAP antagonist). Preferably, the cAMP agonist may be forskolin and/or NKH 477.

Among the above compounds, forskolin may be contained at a concentration of 1 nM to 500 µM, preferably 0.5 µM to 50 µM, more preferably 1 µM to 45 µM, even more preferably 25 µM to 50 µM, and NKH 477 may be contained at a concentration of 0.5 µM to 100 µM. When forskolin and/or NKH 477 are/is contained at a concentration within the above range, it may be preferable in terms of being effective and able to exhibit an effect without toxicity.

In the present disclosure, "histone demethylase inhibitor" serves to inhibit LSD1, an enzyme that selectively demethylates two lysines found in histone H3, and serves to inhibit oxidative deamination of monoamines. The histone demethylase inhibitor may be contained at a concentration of 1 nM to 100 mM

The histone demethylase inhibitor is not limited in the type thereof, but specifically, may be parnate (tranylcypromine), SP2509, Ciclipirox, Daminozide, GSK J1, GSK J2, GSK J4, GSK J5, GSK LSD1, (R)-2-hydroxyglutaric acid, IOX1, JIB04, NSC636819, 0G-L002, PBIT, RN 1 dihydrochloride, S2101, or TC-E 5002. Preferably, the histone demethylase inhibitor may be parnate (tranylcypromine) and/or SP2509.

Among the histone demethylase inhibitors, parnate may be contained at a concentration of 1 nM to 1 mM, preferably 0.5 µM to 500 µM, more preferably 1 µM to 100 µM, even more preferably 10 µM to 20 µM, and SP2509 may be contained at a concentration of 1 nM to 100 nM. When parnate (tranylcypromine) and/or SP2509 are/is contained at a concentration within the above range, it may be preferable in terms of being effective and being able to exhibit an effect without toxicity.

In the present disclosure, it is more preferable that the first chemical cocktail and/or the second chemical cocktail further contain(s) a RAR agonist. In this case, protein factors having neuroprotective and neuroregenerative functions are better expressed. In the present disclosure, the RAR agonist is not limited in the kind thereof, but specifically, may be TTNPB (4-[(E)-2-(5,6,7,8-tetrahydro-5,5,8,8-tetramethyl-2-naphthalenyl)-1-propenyl]benzoic acid; or arotinoid acid).

The RAR agonist may be contained at a concentration of 1 nM to 500 µM, preferably 10 nM to 2 µM, more preferably 20 µM to 1.5 µM, even more preferably 1 µM to 2 µM. When the RAR agonist is contained at a concentration within the above range, it may be preferable in terms of being effective and being able to exhibit an effect without toxicity. In the present disclosure, the somatic cells are not limited in the kind thereof, but may be fibroblasts isolated from various tissues, fully differentiated somatic cells including blood cells or adipocytes, and/or adult stem cells (or somatic stem cells) present in umbilical cord, placenta, umbilical cord blood, bone marrow, blood, fat, etc.

The present invention also provides a method for producing glia-like cells that are differentiated from somatic cells, the method including a differentiation induction step of inducing differentiation of the somatic cells by treatment with a first chemical cocktail containing a histone deacetylase inhibitor, a GSK inhibitor, an ALK-5 kinase inhibitor, a cAMP agonist, a histone demethylase inhibitor and an RAR agonist.

The differentiation induction step may be a step of inducing differentiation of the somatic cells by treatment with a first chemical cocktail containing a histone deacetylase inhibitor, a GSK inhibitor, an ALK-5 kinase inhibitor, a cAMP agonist and a histone demethylase inhibitor. In the present disclosure, any glia-like cells that are differentiated from somatic cells, produced by the method including the differentiation induction step, may be used without limitation, but it may be more preferable that the method further includes a re-culture step after the differentiation induction step.

In addition, the method of the present disclosure includes a differentiation induction step of inducing differentiation of the somatic cells by treatment with a first chemical cocktail containing a histone deacetylase inhibitor, a GSK inhibitor, an ALK-5 kinase inhibitor, a cAMP agonist and a histone demethylase inhibitor, and may further include, between the differentiation induction step and the re-culture step, a maturation step of maturing the somatic cells, treated with the first chemical cocktail, by further treatment with a second chemical cocktail containing a GSK inhibitor, an ALK-5 kinase inhibitor and a cAMP agonist. Where the maturation step is included, the period (days) of the maturation step may preferably be equal to or shorter than the period (days) of the differentiation induction step.

The present disclosure may also provide a method for producing glia-like cells, the method including a differentiation induction step of inducing differentiation of the somatic cells by treatment with a first chemical cocktail containing a histone deacetylase inhibitor, a GSK inhibitor, an ALK-5 kinase inhibitor, a cAMP agonist and a histone demethylase inhibitor.

In addition, the method of the present disclosure may further include a re-culture step after the differentiation induction step. Where the re-culture step is included, it is more preferable because it is possible to provide glia-like cells that may be stably passaged in a culture medium and/or medium free of low-molecular compounds.

The production method of the present disclosure may further include, between the differentiation induction step and the re-culture step, a maturation step of maturing the somatic cells, treated with the first chemical cocktail, by further treatment with a second chemical cocktail containing a GSK inhibitor, an ALK-5 kinase inhibitor and a cAMP agonist. Where the maturation step is included, the period (days) of the maturation step may preferably be equal to or shorter than the period (days) of the differentiation induction step.

The present disclosure also provides a method for producing glia-like cells that are differentiated from somatic cells, the method further including: a maturation step of maturing the cells after the differentiation induction step; and a re-culture step after the differentiation induction step or after the differentiation induction step and the maturation step.

The differentiation induction step serves to activate cells (e.g., fibroblasts, etc.) to be differentiated, thus allowing the cells to enter the stage of differentiated cells (e.g., novel glia-like cells, etc.). The differentiation induction step may be performed for at least 3 days, preferably 3 to 18 days.

The maturation step means performing culture so that the cells that entered the stage of differentiated cells may fully have the activity of the differentiated cells. The differentiation induction step/maturation step may be performed for 3 days or more, but when the differentiation induction step is adjusted to a longer period, it may replace the maturation step, and in this case, the maturation step may be omitted.

The re-culture step is necessary to ensure that the differentiated cells may be maintained as stable cells that may be passaged without low-molecular compounds. The re-culture step may include a step of further culturing the differentiation-induced cells for at least 3 days, and the re-cultured cells may be stably passaged so that long-term culture is possible. However, a re-culture period of 3 to 12 days may be more preferable. The re-culture step includes a step of culturing the cells in a medium in a state in which Matrigel was removed. More specifically, the re-culture step refers to a step of additionally culturing the glia-like cells differentiation-induced from the somatic cells in the absence of small-molecular compounds and Matrigel (BD Bioscience).

More specifically, the re-culture step may be performed through the following method, but is not limited thereto. The cells subjected to the differentiation induction step from somatic cells and/or the maturation step are washed twice with 1X PBS, and 3 ml of warm accutase^{®} solution (stored in a 37°C constant-temperature water bath) is added thereto in an incubator for 15 minutes. After 15 minutes, whether or not the cells were separated is observed under a bright-field microscope. Complete DMEM is added thereto, and the solution is gently pipetted to separate the cell-containing Matrigel layer containing cells from the 60-mm dish. The solution is passed through a cell strainer to remove the Matrigel. The filtered cells are centrifuged at 1000 rpm for 5 min at RT. The supernatant is discarded, and the cell pellet is gently resuspended in a complete Schwann cell medium (Science Research Laboratory), and added to a fresh plate without a coating such as Matrigel. While the plate having the cells added thereto is stored in an incubator at 37°C, culture is performed for a total of 3 to 12 days, preferably 6 to 12 days. During culture, the medium is replaced every 3 days.

The differentiation induction step and/or maturation step before the re-culture step may include Matrigel.

In addition, the re-culture step may include a step of freezing the cells under liquid nitrogen (N₂). That is, where the cells are to be stored for a long period of 12 days or more, the cells may be freeze-dried and stored for a long period, instead of the method of continuously culturing the cells, and if necessary, the cells may be thawed and cultured again in a re-culture medium.

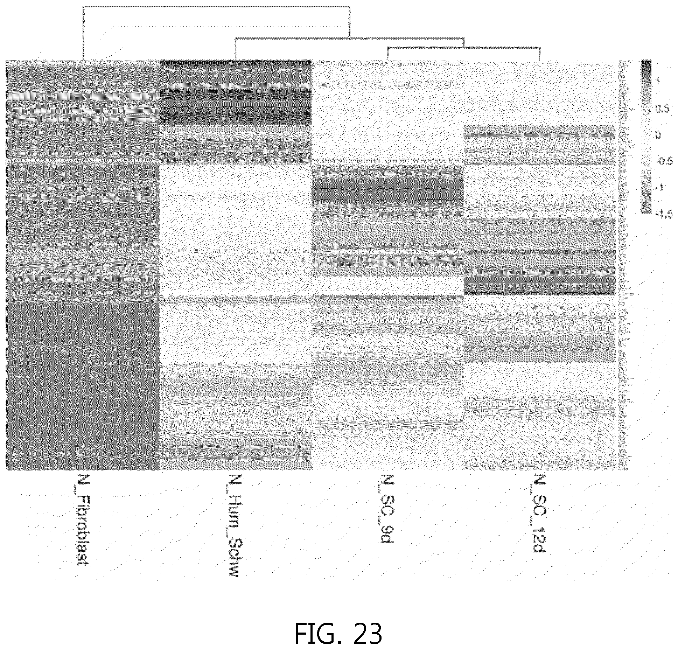

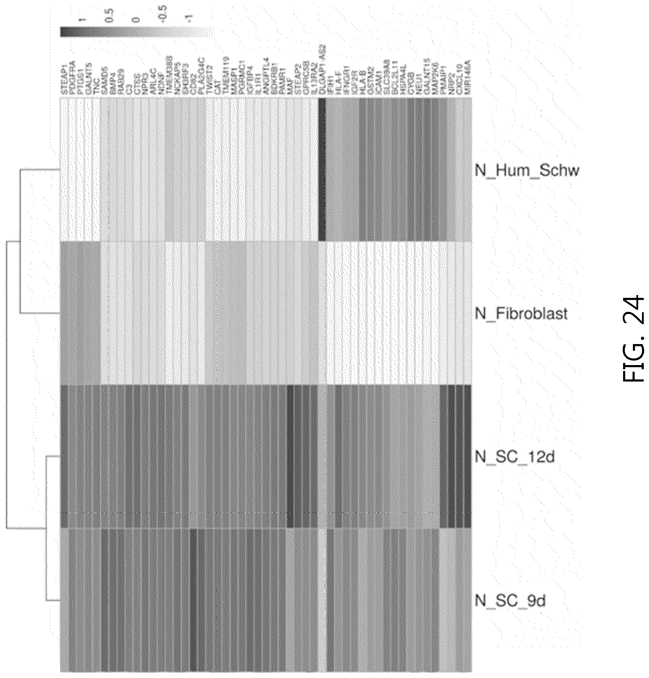

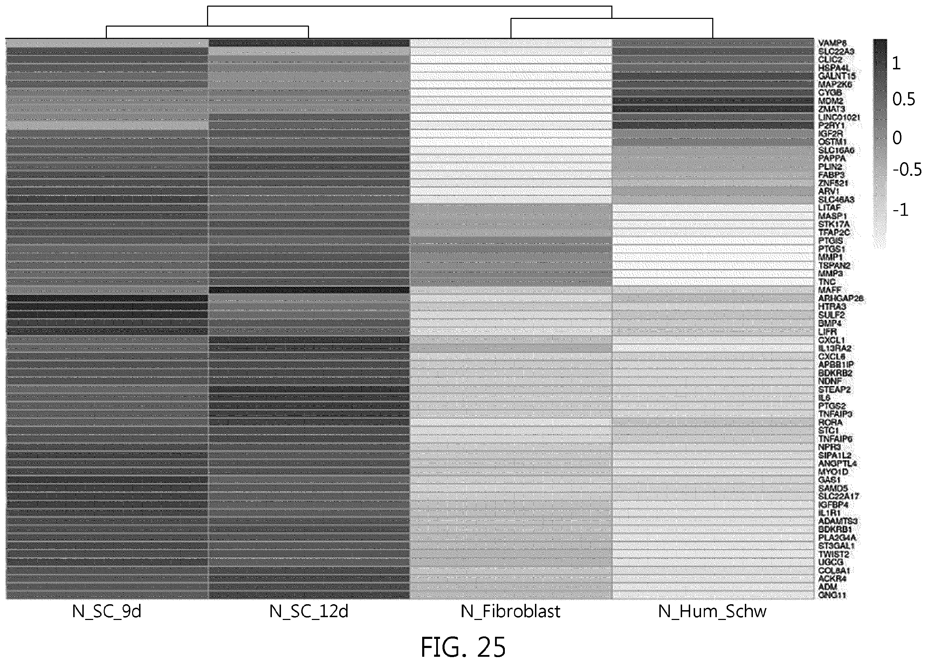

The novel glia-like cells of the present disclosure are obtained by differentiation from somatic cells, and the efficiency of differentiation may be determined based on the degree of cells having a morphology characterized by glia-like cells, or the degree of secretion of protein factors overexpressed in glia-like cells characterized by HGF, MIF or BDNF, or marker genes expressed in each type of cells. The markers may be classified into FBLN5, DKK1, FBN1, PRRX1 and/or ECM1, which are/is expressed in fibroblasts, and MBP, NDGR1, GALC, GFAP and/or MPZ, which are/is expressed in neuroglial cells. When somatic cells are differentiated into glia-like cells with excellent efficiency, the expression level of FBLN5, DKK1, FBN1, PRRX1 and/or ECM1, which are/is better expressed in fibroblasts, decreases, and the expression level of the neuroglial cell markers MBP, NDGR1, GALC, GFAP and/or MPZ increase (see FIGS. 23 to 27).

The present disclosure also provides a cell therapy product for treating neurological disorder containing, as an active ingredient, the above-described novel glia-like cells that are differentiated from somatic cells. The neurological disorder refers to a neurodegenerative disorder, a neurological disorder caused by inheritance, a disorder caused by nerve injury, and the like. Examples of the neurodegenerative disorder include amyotrophic lateral sclerosis (ALS), frontal temporal dementia (FTD), Alzheimer's disease, Parkinson's disease, senile dementia, Huntington's disease, and the like; examples of the neurological disorder caused by inheritance include Gilles de la Tourette's syndrome, congenital motor and sensory neuropathy, Charcoal-Marie-Tooth disease (CMT), familial dysautonomia, Lowe syndrome, Rett syndrome, cerebral autosomal dominant arteriopathy with subcortical infarcts and leukoencephalopathy (CADASIL), tuberous sclerosis, Ataxia telangiectasia, neurofibromatosis, and the like; and examples of the neurological disorder caused by nerve injury include multiple sclerosis, Guillain-Barre syndrome (GBS), acute inflammatory demyelinating neuropathy (polyradiculopathy type), schwannomatosis and chronic inflammatory demyelinating polyneuropathy (CIDP), diabetic neuropathy, neuropathy caused by anticancer drugs, peripheral nerve amputation caused by trauma, spinal cord injury, and various nerve injury diseases including peripheral and central nervous system tissue diseases such as glaucoma.

In addition, the present disclosure may provide a method for preventing and treating neurological disorder, the method including a step of administering the above-described glia-like cells that are differentiated from somatic cells to a subject with neurological disorder.

The cell therapy product according to the present disclosure may contain a pharmaceutically effective amount of the glia-like cells or may further contain one or more pharmaceutically acceptable carriers, excipients, or diluents. Here, the pharmaceutically effective amount refers to an amount sufficient to prevent, ameliorate and treat symptoms of an immune disease.

The number of the glia-like cells contained as an active ingredient according to the present disclosure may be 1×10² to 1×10¹⁵, and the pharmaceutically effective amount may appropriately change depending on the degree of symptoms of immune disease, the patient's age, weight, health condition and sex, the route of administration, and the period of treatment.

As used herein, the term "pharmaceutically acceptable" refers to a composition that is physiologically acceptable and, when administered to humans and/or mammals, generally does not cause gastrointestinal disorders, allergic responses such as dizziness, or similar responses. Examples of the carriers, excipients and diluents include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methylhydroxybenzoate, propylhydroxybenzoate, talc, magnesium stearate, and mineral oil. In addition, the cell therapy product may further contain a filler, an anti-aggregating agent, a lubricant, a wetting agent, a fragrance, an emulsifier a preservative, and the like.

In addition, the cell therapy product of the present disclosure may be formulated using a method known in the art so as to provide quick, sustained or delayed release of the active ingredient after administration to humans and/or mammals. The formulation may be in the form of powder, granule, tablet, emulsion, syrup, aerosol, soft or hard gelatin capsule, sterile injection solution, or sterile powder.

In addition, the cell therapy product for treating and/or preventing neurological disorder according to the present disclosure may be administered through various routes including oral, transdermal, subcutaneous, intravenous or intramuscular route, and administered by direct injection into an injured area and/or transplantation through surgery. In addition, the dosage of the active ingredient may be appropriately selected depending on various factors such as the route of administration, the patient's age, sex, weight, and disease severity, and the composition for preventing or treating immune disease according to the present disclosure may be administered in combination with a known compound having an effect of preventing, ameliorating, or treating symptoms of the immune disease.

The present disclosure reports the development of the chemical-based conversion of human fibroblasts into novel glia-like cells having neuroregenerative, neurorepairing and neuroprotective cell functions as well as properties very similar to those Schwann cells. The present inventors have determined that chemical cocktails containing a histone deacetylase inhibitor, a GSK inhibitor, an ALK-5 kinase inhibitor, a cAMP agonist and a histone dimethylase inhibitor are important for this conversion. In order to specify and identify various aspects of such a novel neuron type and to evaluate the functionality thereof, the following preparation examples, examples, and the like are provided. However, the scope of the present disclosure is not limited to the following examples.

Preparation Examples: Production of Glia-Like Cells (Cells Having Neurorepairing and Neuroprotective Functions; Neurorepair-and-protection cells; Hereinafter Referred to as "Glia-Like Cells") under various protocols

### Production Example 1: Cell Culture

Human foreskin fibroblasts (SCC058, Millipore) were cultured in high-glucose DMEM (Welgene) supplemented with 10% FBS and 1% penicillin and streptomycin (Welgene). Human Schwann cells were cultured in a Schwann cell medium composed of a Schwann cell basal medium (ScienCell) containing growth additives (Science) and 1% penicillin and streptomycin (Welgene). Human neuron cell line NSC34 was cultured in high-glucose DMEM (Welgene) supplemented with 10% FBS and 1% penicillin and streptomycin (Welgene).

### Preparation Example 2: Preparation of Media Used for Differentiation

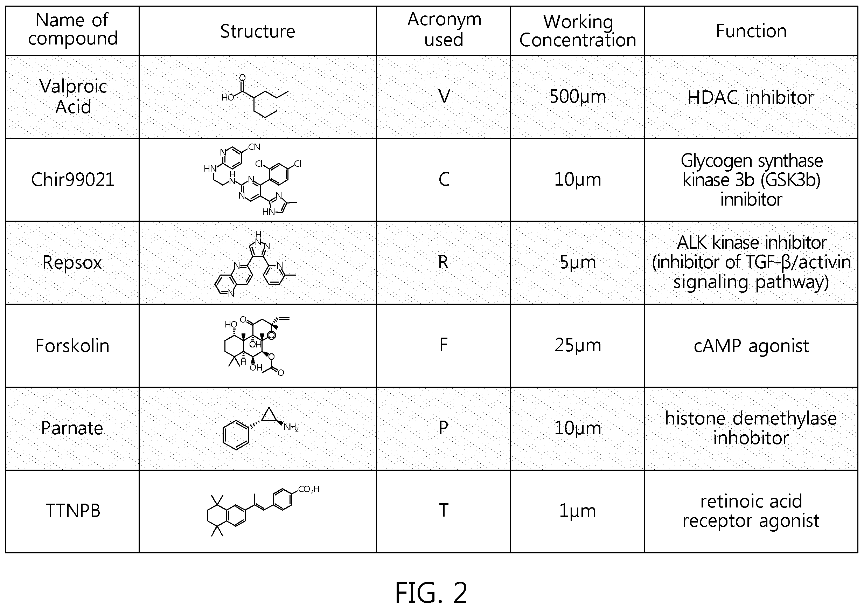

Reprogramming medium (RM): knockout DMEM (Gibco), 15% knockout serum replacement, 5% FBS (Gibco), 1% Glutamax (Gibco), 1% nonessential amino acids (Gibco), 0.1 mM β-mercaptoethanol (Sigma) and 1X penicillin/streptomycin, first chemical cocktail (Table 1, or FIG. 2: 500 µM valproic acid (V), 10 µM CHIR99021 (C); 5 µM RepSox (R); 25 µM forskolin (F); 10 µM tranylcypromine (P); 1 µM TTNPB (T)), (all small molecular compounds used in the cocktail were obtained from Medchemexpress).

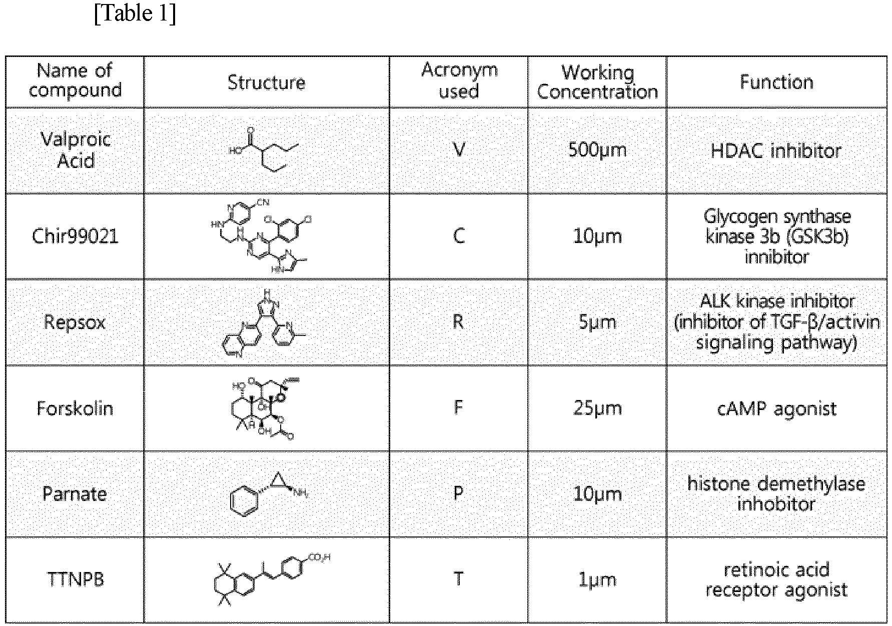

Maturation medium (MM): containing knockout DMEM (Gibco), 15% knockout serum replacement, 5% FBS (Gibco), 1% Glutamax (Gibco), 1% nonessential amino acid (Gibco), 0.1 mM β-mercaptoethanol (Sigma) and 1× penicillin/streptomycin), a second chemical cocktail composed of 10 µM CHIR99021 (C); 5 µM RepSox (R); 25 µM forskolin (F).

Reculture medium: cells were cultured in a Schwann cell medium consisting of a Schwann cell basal medium (ScienCell) containing growth additives (Science) and 1% penicillin and streptomycin (Welgene).

### Preparation Example 3: Preparation of Medium Used for Differentiation

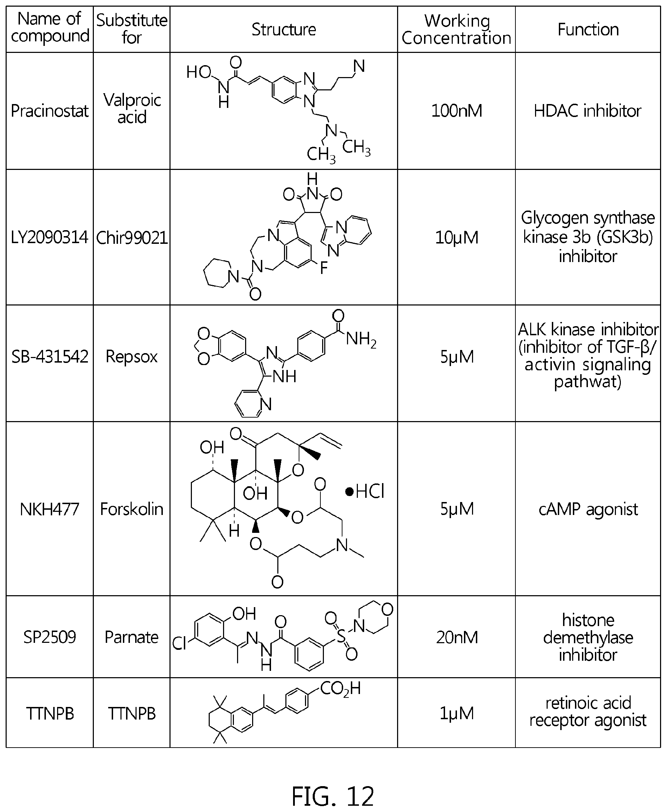

A medium was prepared in the same manner as in Preparation Example 2, except that the compounds used in the first chemical cocktail shown in Table 1 or FIG. 2 were replaced with the compounds shown in Table 2 or FIG. 12. The second chemical cocktail was prepared in the same manner as in the maturation medium preparation method of Preparation Example 2, except that LY2090314, SB-431542 and NKH477 were included as shown in Table 2 below.

**[Table 2]**

| Name of compound | Substitute for | Structure | Working Concentration | Function |
|---|---|---|---|---|
| Pracinostat | Valproic acid | 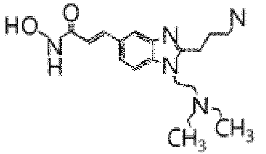 | 100nM | HDAC inhibitor |
| LY2090314 | Chir99021 | 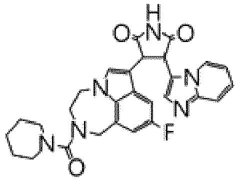 | 10µM | Glycogen synthase kinase 3b (GSK3b) inhibitor |
| SB-431542 | Repsox | 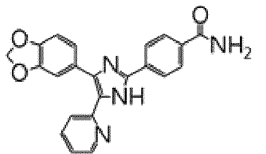 | 5µM | ALK kinase inhibitor (inhibitor of TGF-β/ activin signaling pathwat) |
| NKH477 | Forskolin | 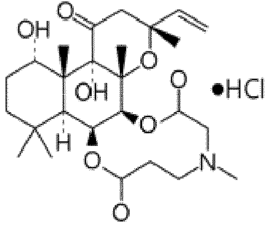 | 5µM | cAMP agonist |
| SP2509 | Pamate | 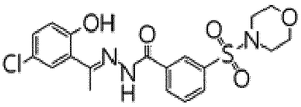 | 20nM | histone demethylase inhibitor |
| TTNPB | TTNPB | 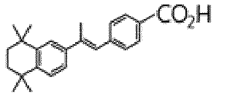 | 1µM | retinoic acid receptor agonist |

### Examples: Production of Glia-Like Cells According to Protocols I, II and III

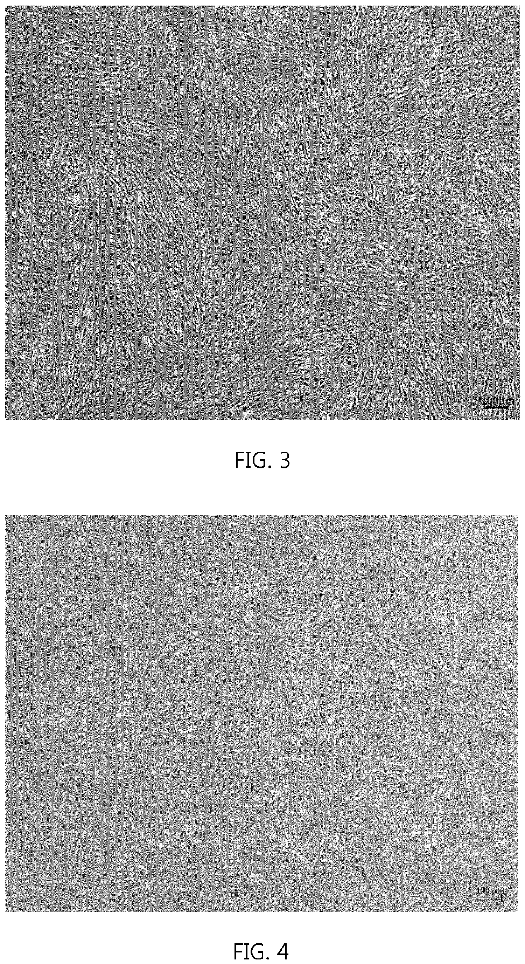

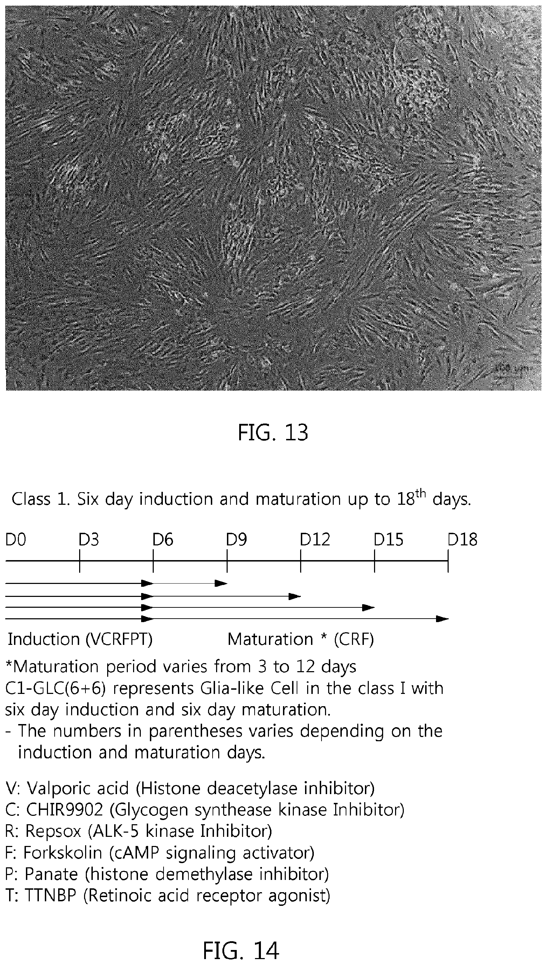

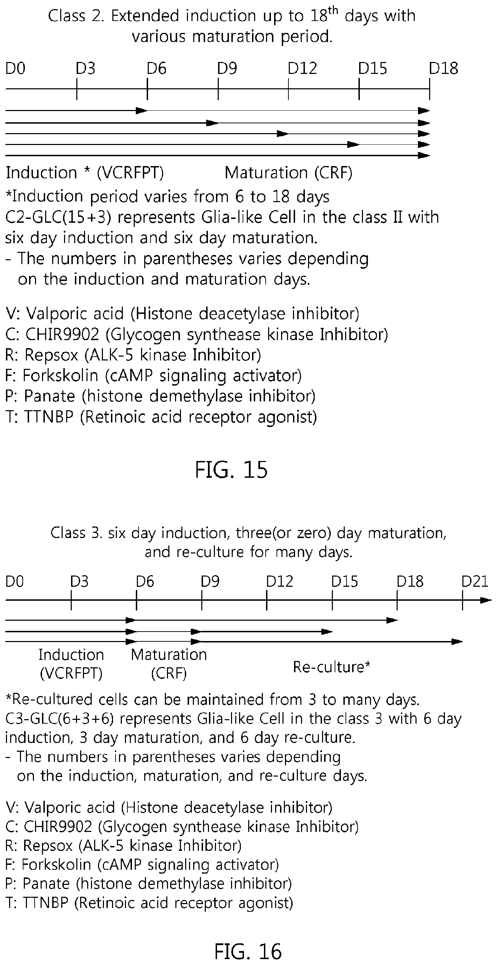

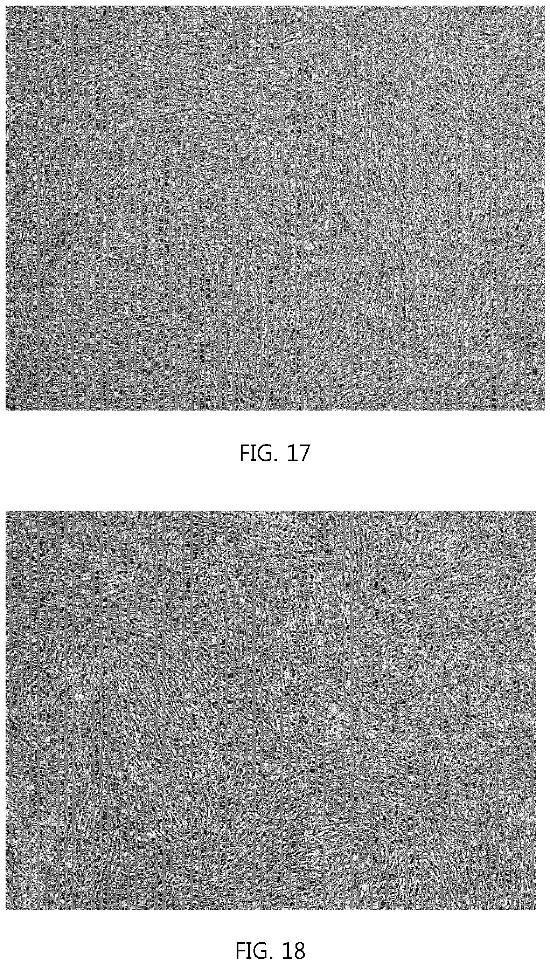

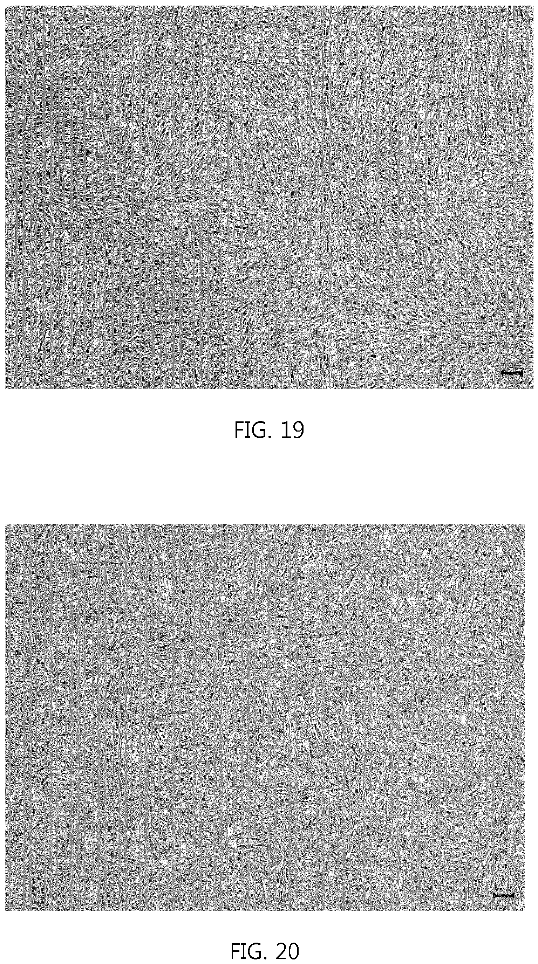

After the initial conversion was confirmed as shown in FIG. 3, the present inventors used several different conversion protocols and monitored conversion efficiency. According to the conversion conditions, glia-like cells are classified into three protocol cell types, that is, protocol I (FIG. 14), protocol II (FIG. 15) and protocol III (FIG. 16) glia-like cell types (FIGS. 14 to 16). Protocol 1 is characterized in that the induction time is fixed while the maturation time is changed; protocol 2 is characterized in that the induction time is changed; and protocol 3 is characterized in that differentiated cells are recultured without chemical compounds. The glia-like cells obtained by conversion according to protocol I, protocol II and protocol III are referred to as Cl-glia-like cells (C1-GLC), C2-glia-like cells (C2-GLC) and C3-glia-like cells (C3- GLC), respectively. As a result of examining the morphology of each of these cells through microscopic analysis (FIGS. 18 to 20 ), it could be seen that these cells showed a morphology different from that of the original human foreskin fibroblasts (FIG. 17) used for differentiation, but had a morphology similar to that of human Schwann cells (FIG. 21).

### Example 1: Protocol I (Production of C1-Glia-Like Cells):

Human foreskin fibroblasts were trypsinized and resuspended in RM. The cells were dispensed into a Matrigel-coated 60-mm tissue culture plate (pre-coated with 1:100 Matrigel (BD Biosciences) for 2 hours at room temperature) at a density of 6×10⁵ cells. Culturing by the differentiation induction step was performed while the culture medium was replaced with a fresh RM medium every 3 days. Induction by the differentiation induction step was continued until day 6. On day 6 of induction, the culture medium was replaced with MM, and culturing by maturation was then performed for an additional 3 to 12 days.

### Example 1-1: Cl-Glia-Like Cells(6+3)

Novel glia-like cells that are differentiated from somatic cells were produced in the same manner as in Example 1, except that the culturing by the maturation step was performed for 3 days.

### Example 1-2: Cl-Glia-Like Cells(6+6)

Novel glia-like cells that are differentiated from somatic cells were produced in the same manner as in Example 1, except that the culturing by the maturation step was performed for 6 days.

### Example 1-3: Cl-Glia-Like Cells(6+9)

Novel glia-like cells that are differentiated from somatic cells were produced in the same manner as in Example 1, except that the culturing by the maturation step was performed for 9 days.

### Example 1-4: Cl-Glia-Like Cells(6+12)

Novel glia-like cells that are differentiated from somatic cells were produced in the same manner as in Example 1, except that the culturing by the maturation step was performed for 12 days.

### Example 2: Protocol II (Production of C2-Glia-Like Cells):

Human foreskin fibroblasts were trypsinized and resuspended in RM. The cells were dispensed into a Matrigel-coated 60-mm tissue culture plate (pre-coated with 1:100 Matrigel (BD Biosciences) for 2 hours at room temperature) at a density of 6×10⁵ cells. Culturing by the differentiation induction step was performed while the culture medium was replaced with a fresh RM medium every 3 days. The culturing by the differentiation induction step was continued for 6 to 15 days, and the induction period was immediately followed by a fixed maturation period of 3 days. Alternatively, the differentiation induction step was performed for 18 days without the maturation period.

### Example 2-1: C2-Glia-Like Cells(6+12)

Novel glia-like cells that are differentiated from somatic cells were produced in the same manner as in Example 2, except that the culturing by the differentiation induction step was performed for 6 days and the culturing by the maturation step was performed for 12 days.

### Example 2-2: C2-Glia-Like Cells(9+9)

Novel glia-like cells that are differentiated from somatic cells were produced in the same manner as in Example 2, except that the culturing by the differentiation induction step was performed for 9 days and the culturing by the maturation step was performed for 9 days.

### Example 2-3: C2-Glia-Like Cells(12+6)

Novel glia-like cells that are differentiated from somatic cells were produced in the same manner as in Example 2, except that the culturing by the differentiation induction step was performed for 12 days and the culturing by the maturation step was performed for 6 days.

### Example 2-4: C2-Glia-Like Cells(15+3)

Novel glia-like cells that are differentiated from somatic cells were produced in the same manner as in Example 2, except that the culturing by the differentiation induction step was performed for 15 days and the culturing by the maturation step was performed for 3 days.

### Example 2-5: C2-Glia-Like Cells(18+0)

Novel glia-like cells that are differentiated from somatic cells were produced in the same manner as in Example 2, except that the culturing by the differentiation induction step was performed for 18 days and the culturing by the maturation step was set to 0 day.

### Example 3: Protocol III (Production of C3-Glia-Like Cells)

Human foreskin fibroblasts were trypsinized and resuspended in RM. The cells were dispensed into a Matrigel-coated 60-mm tissue culture plate (pre-coated with 1:100 Matrigel (BD Biosciences) for 2 hours at room temperature) at a density of 6×10⁵ cells. The culture medium is replaced with a fresh RM medium every 3 days. The induction was continued until day 6. On day 6 of the induction, the culture medium was replaced with MM, and culturing by the maturation step was performed for an additional 3 days, or the maturation step was omitted. After completion of the culturing by the differentiation induction step or the maturation step, the cells were harvested through treatment with an accutase cell detachment solution (Millipore), and the harvested cells were resuspended in a Schwann cell culture medium and dispensed into a fresh tissue culture plate. The cells were grown for an additional 6 to 12 days. The culture medium was replaced with a fresh medium every 3 days.

### Example 3-1: C3-Glia-Like Cells(6+0+12)

Novel glia-like cells that are differentiated from somatic cells were produced in the same manner as in Example 3, except that the culturing by the differentiation induction step was performed for 6 days, the culturing by the maturation step was omitted, and then the reculture step was performed for 12 days.

### Example 3-2: C3-Glia-Like Cells(6+3+6)

Novel glia-like cells that are differentiated from somatic cells were produced in the same manner as in Example 3, except that the culturing by the differentiation induction step was performed for 6 days, the culturing by the maturation step was performed for 3 days, and then the reculture step was performed for 6 days.

### Example 3-3: C3-Glia-Like Cells(6+3+12)

Novel glia-like cells that are differentiated from somatic cells were produced in the same manner as in Example 3, except that the culturing by the differentiation induction step was performed for 6 days, the culturing by the maturation step was performed for 6 days, and then the reculture step was performed for 12 days.

### Example 4: Protocol III-1 (Reculture After Freezing of C3-Glia-Like Cells)

The C3-glia-like cells produced according to protocol III were trypsinized after completion of 6 to 12 days of reculture, resuspended in a freezing medium (high-glucose DMEM (Welgene) containing 20% FBS and 10% DMSO), and freeze-stored in liquid N₂ until further use. For use, the vial of the C3-glia-like cells was thawed, and the cells were dispensed in complete Schwann cell culture medium.

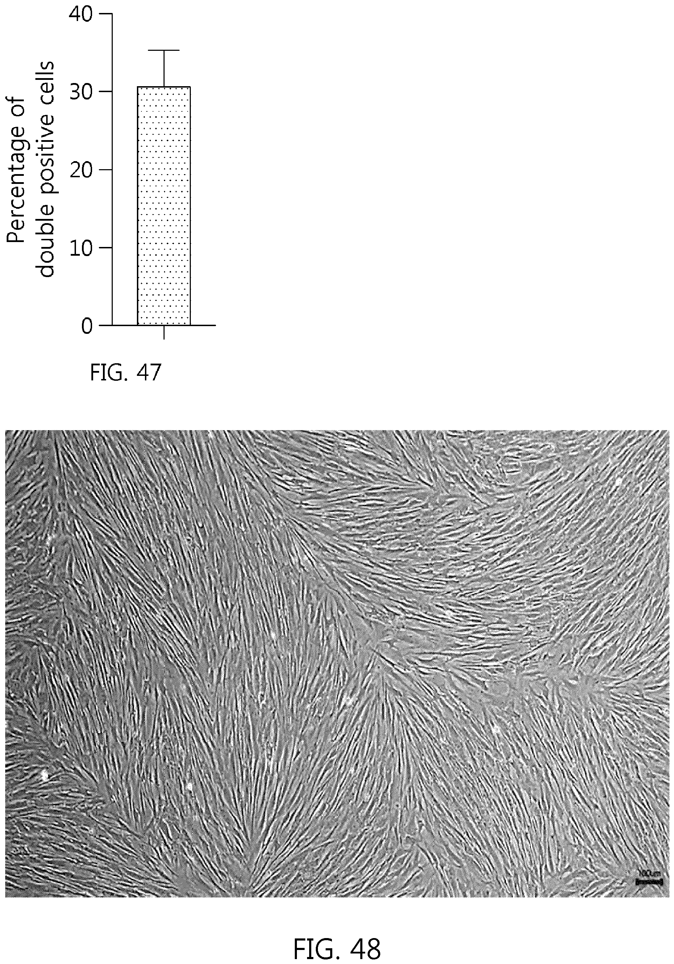

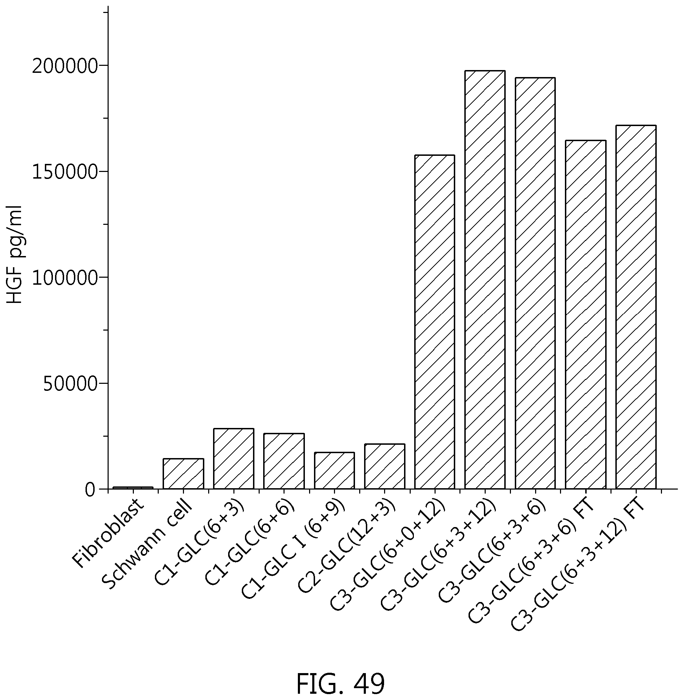

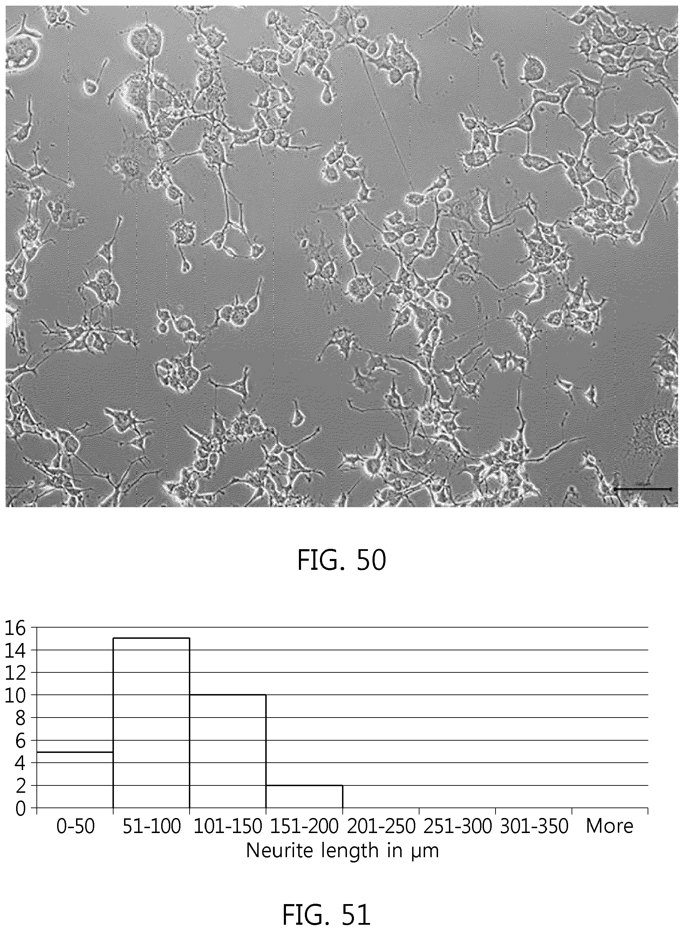

In order to confirm whether the C3-glia-like cells can be safely frozen without loss of functionality, thawed and then re-cultured, the present inventors performed freezing and reculture experiments. After thawing and culturing of the frozen C3-glia-like cells, the present inventors could not observe significant differences in cell morphology (FIG. 48), cytokine release (FIG. 49) and neurite outgrowth promoting ability (FIGS. 50 and 51) from the cells before freezing.

### Experimental Examples

### Experimental Example 1: Direct Differentiation of Fibroblasts into Glia-Like Cells Using Low-Molecular Compounds

With regard to the differentiation of somatic cells into glia-like cells, it has been reported that conventional small molecular compounds that control several important signaling pathways can convert mouse fibroblasts into pluripotent stem cells (CiPSCs). In addition, it has been reported in previous studies that transient activation of pluripotency programs in somatic cells activates these cells. That is, it has been suggested that these activated cells can be differentiated into several lineages by simply exposing them to several growth factors and cytokines. Accordingly, the present inventors attempted to generate a similar activated state using chemicals similar to those used to generate CiPSC cells, and attempted to induce this state into several lineages. The present inventors improved the present invention by assuming that a simple treatment method using these chemicals can lead to an activated state that can be induced into several lineages (FIG. 1).

Therefore, the present inventors treated human fibroblasts with 6 kinds of chemicals, that is, valproic acid (V), Chir99021 (C), Repsox (R), Forskolin (F), Parnate (P) and TTNPB (T) (Table 1 or FIG. 2) in human fibroblast reprogramming medium (RM) for 6 to 18 days, and then converted the cells into glia-like cells by maintaining them in a maturation medium (MM) containing CHIR99021 (C), RepSox (R) and forskolin (F) for additional 3 to 12 days, and recultured the converted cells in Schwann cell culture medium without compounds (FIG. 1). It was confirmed that the glia-like cells thus obtained had a morphology completely different from that of human fibroblasts, but had a spindle-shaped cell morphology having a nuclear/cytoplasmic ratio similar to that of human Schwann cells (FIGS. 3 and 22). More specifically, the glia-like cells are elongate cells having a length of 50 to 300 vL and a diameter of 15 to 50 vL, and taper from the bulging center to both ends or several ends (<10), and in some cases, this end portion has a long thread shape of about 1 micron in width.

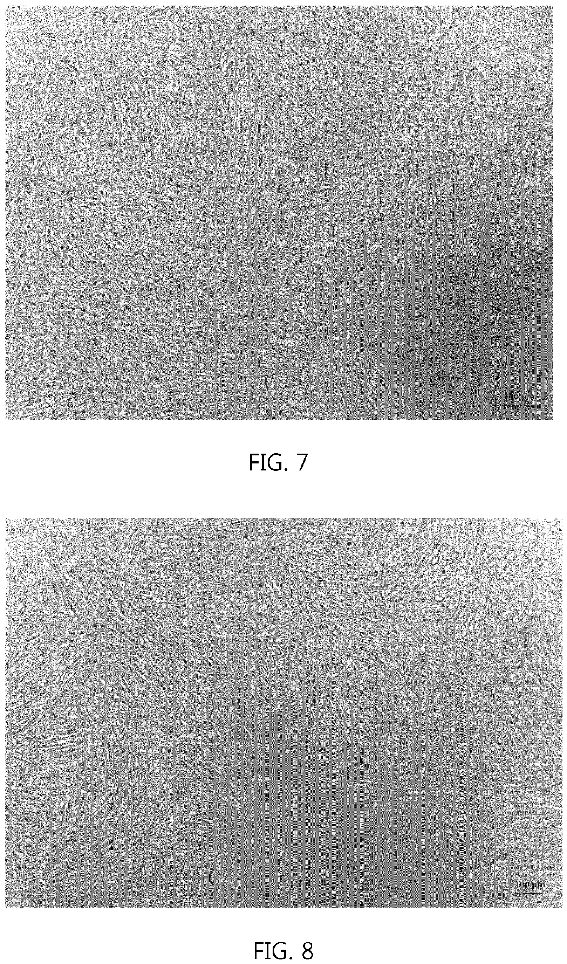

Glia-like cells were produced through an induction step, a maturation step, and a reculture step (FIG. 1). For each step, cells were maintained in induction medium, maturation medium or reculture medium, and at this time, even when the maintenance time was changed in various ways (3 to 15 days, FIGS. 14 to 16), there was no significant change in differentiation into the glia-like cells (FIGS. 8 to 20).

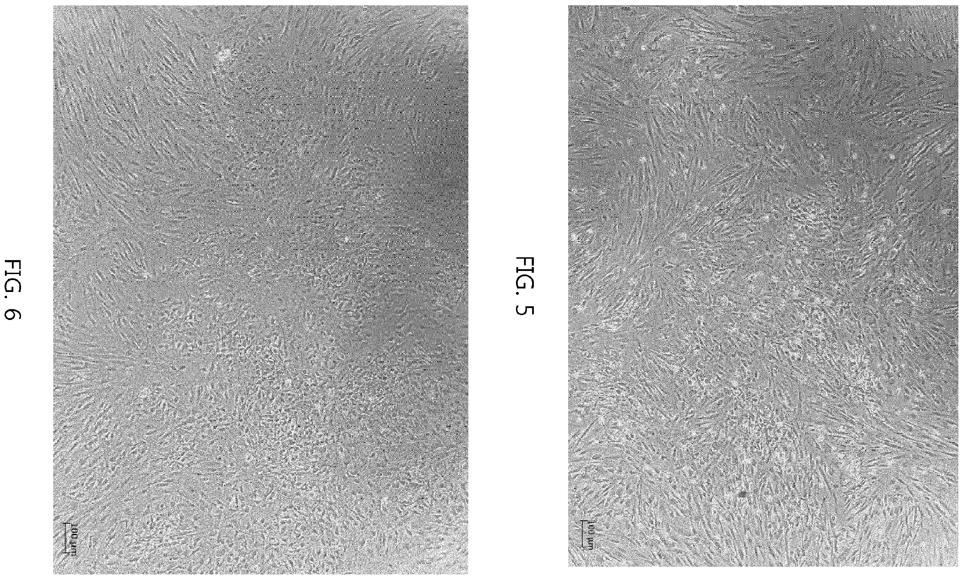

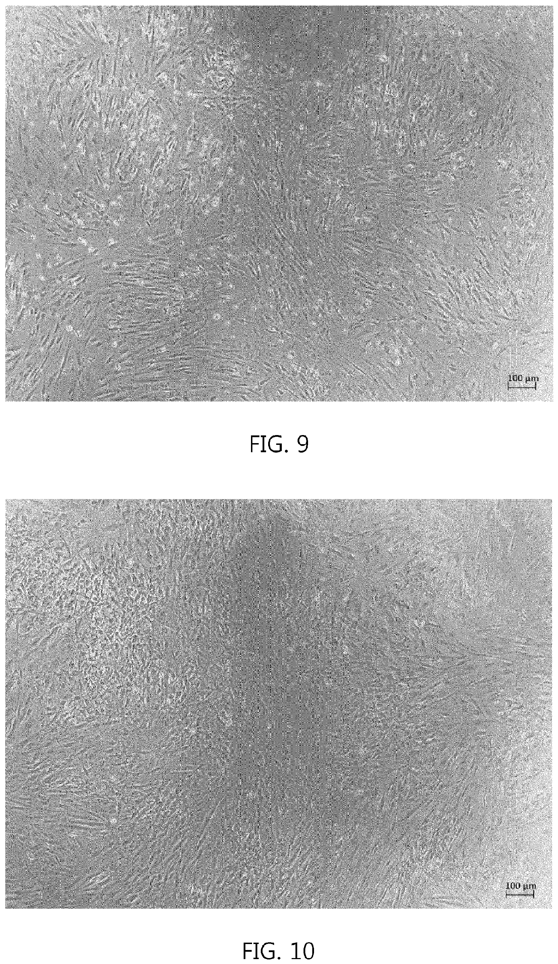

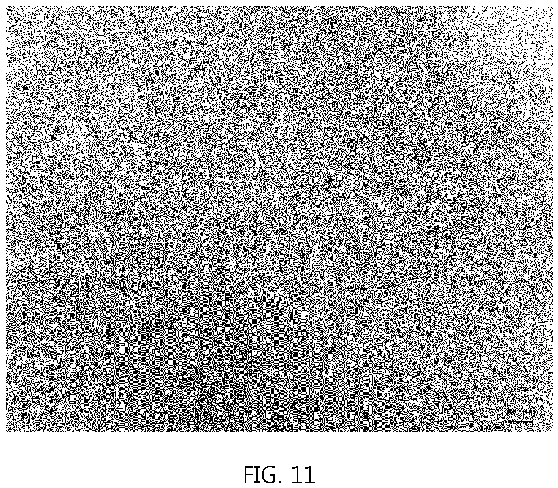

Whether or not the differentiation was successful was determined based on this cell morphology (FIG. 3), and the present inventors examined the minimum necessary compounds of the cocktail. To this end, the present inventors removed one chemical at a time from the basic chemical cocktail (see FIGS. 4 to 11), and used microscopic bright field images to evaluate the resemblance between the glia-like cells obtained by conversion using the six compounds (V, C, R, F, P and T) (FIG. 3). Consequently, it was observed that, when any one of V, C, R, and P was removed, the efficiency of differentiation into glia-like cells decreased (FIGS. 4 to 9). In addition, the present inventors observed that the removal of forskolin had the most severe effect on the conversion yield (FIG. 8). It was confirmed that, when parnate and TTNPB were removed, the conversion yield decreased, but conversion was possible (FIG. 10), and removal of TTNPB had minimal or little effect (FIG. 11). Taking these observations together, the present inventors selected V, C, R, F and P as the first chemical cocktail, because these chemicals correspond to necessary and sufficient conditions to allow fibroblasts to exhibit the morphology of glia-like cells.

In addition, human fibroblasts treated with the chemical cocktail of Preparation Example 2 (FIG. 12) also exhibited the same morphological change as fibroblasts treated with the chemical cocktail of Preparation Example 1, and were differentiated into glia-like cells very similar to those obtained through the chemical cocktail of Preparation Example 1 (FIG. 13).

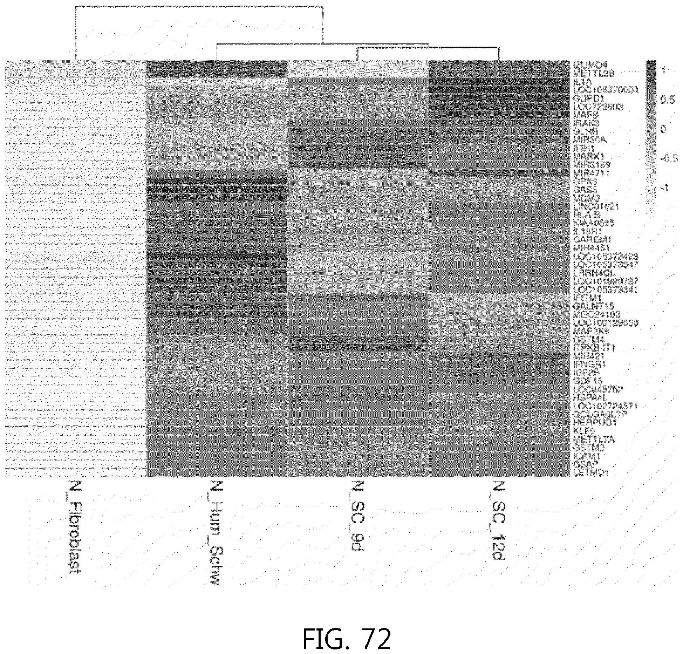

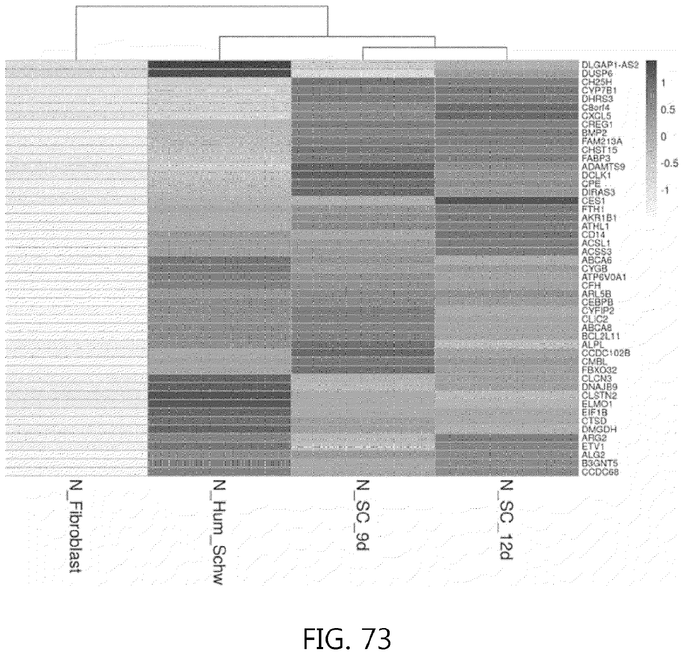

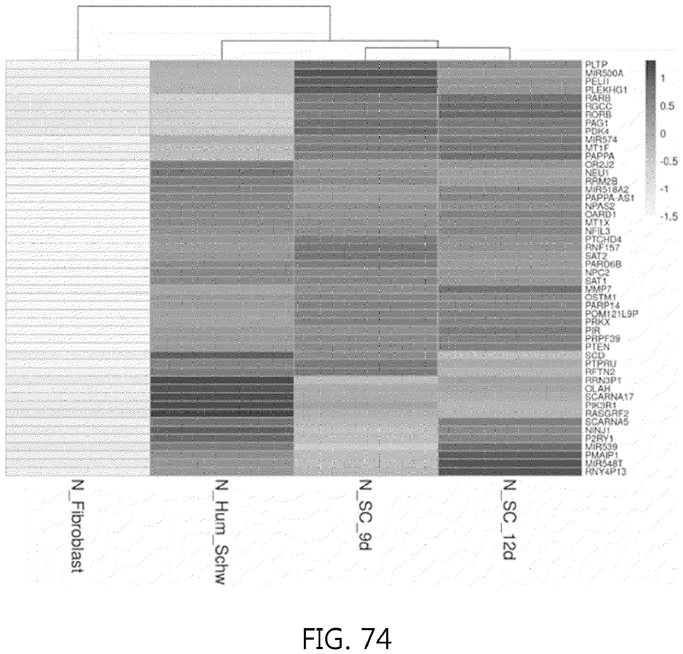

### Experimental Example 2: Microarray (FIGS. 23 to 27 and FIGS. 71 to 74)

Total RNA treated with DNase I was extracted using the RNeasy Mini kit (QIAGEN, CA, USA) as mentioned above. Hybridization to GeneChip Mouse 2.0 ST arrays (Affymetrix, CA, USA) was performed. The data obtained were summarized and normalized using a robust multi-average method conducted by Affymetrix® Power Tools (Affymetrix, CA, USA).

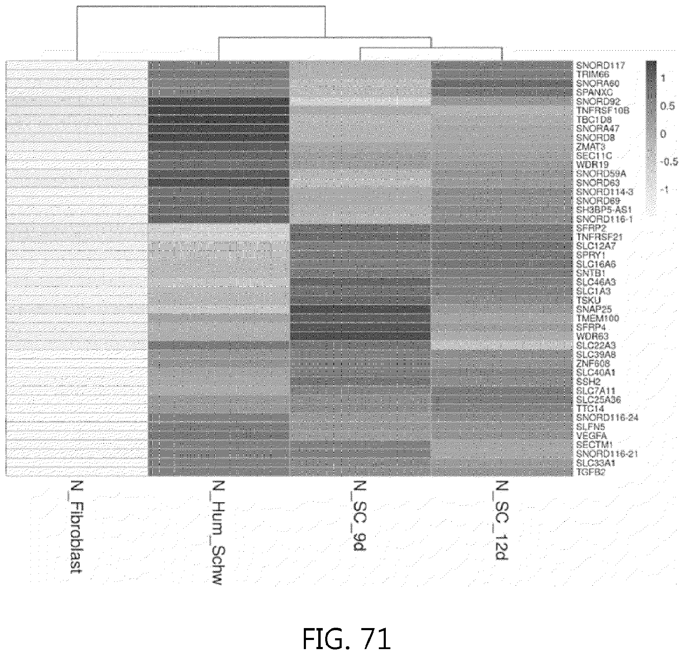

For gene clustering, genes having increased expression in each type of cells, selected based on the microarray results of human Schwann cells, astrocytes and oligodendroglias contained in the GSE database (GSE87385), and genes showing at least doubled expression in the microarray results of the fibroblasts, glia-like cells and Schwann cells used in this experiment, were comparatively analyzed using Venny2.1 (http://bioinfogp.cnb.csic.es/tools/venny), and genes having increased expression in common were selected from the two groups. Gene clustering was performed using ClustVis (https://biit.cs.ut.ee/clustvis/), and a heatmap was drawn (see FIGS. 23 to 25; FIG. 23 is shown in more detail in FIGS. 71 to 74).

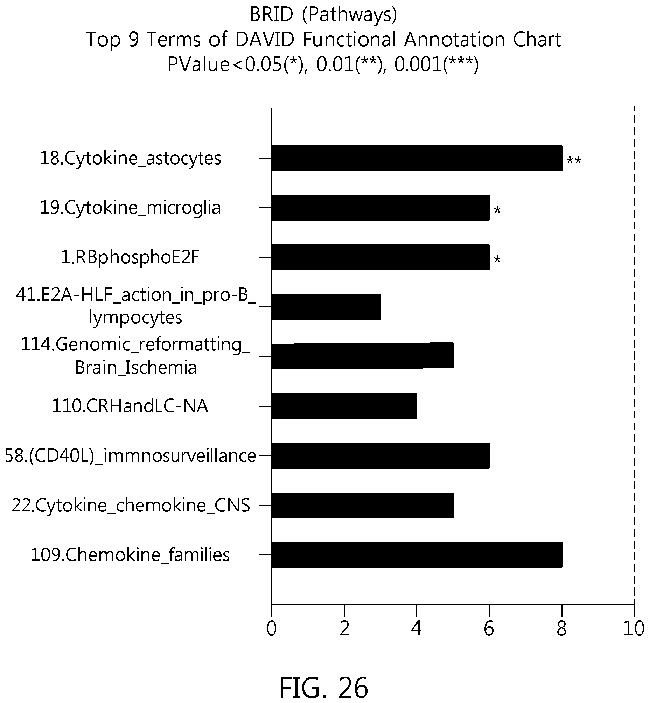

In addition, the analysis results were exported for gene-level analysis and differentially expressed gene (DEG) analysis was performed. The statistical significance of the expression data was determined using fold change. For the DEG set, hierarchical cluster analysis was performed using complete linkage and Euclidean distance as a measure of similarity. Gene-Enrichment and Functional Annotation analysis for a list of significant probes was performed using Gene Ontology (http://geneontology.org) and KEGG (http://kegg.jp) (FIG. 26). Data analysis and visualization of differentially expressed genes were all performed using R 3.0.2 (www.r-project.org).

The result values for the experiment conducted in Experimental Example 2 are shown in FIGS. 23-25.

Looking at the results in FIGS. 23 to 25, it could be confirmed that genes known to be overexpressed in human Schwann cells were not expressed in the fibroblasts used in this experiment, but were mostly expressed in the human Schwann cells and glia-like cells used in this experiment. In addition, it could be confirmed that genes known to be overexpressed in human astrocytes were not expressed in the fibroblasts used in this experiment, and were partially expressed in the human Schwann cells used in this experiment, but were mostly expressed in the glia-like cells. In addition, it could be confirmed that genes known to be overexpressed in human oligodendroglias were not expressed in the fibroblasts used in this experiment, and were partially expressed in the human Schwann cells used in this experiment, but were mostly expressed in the glia-like cells.

Looking at the results in FIG. 26, it can be confirmed that genes expressed in the glia-like cells were increasingly expressed in neuroglial cells such as astrocytes or microglias.

Looking at the results in FIG. 27, it can be confirmed that the expression of the neuroglial marker genes MBP, GFAP, NDRG1, GALC, MPZ, etc. increased in glia-like cells and human Schwann cells, but the expression of FBN1, FBLN5, PRRX1, ECM1, DKK1, and the like, which are marker genes characteristic of fibroblasts, significantly decreased in glia-like cells and human Schwann cells.

### Experimental Example 3: Immunostaining

After the induction step and/or the maturation step or the reculture step, glia-like cells (GLC) derived from human fibroblasts were harvested, washed twice with 1× PBS (Welgene), fixed with 4% paraformaldehyde (Sigma-Aldrich) for 10 minutes, then permeabilized with 0.25% Triton X-100 (USB Corporation) in PBS for 10 minutes at 22°C, and washed twice with PBS for 5 minutes each. Next, the cells were blocked with a blocking solution containing 1% BSA (Amresco), 22.52 mg/mL glycine (Affymetrix) and 0.1% Tween 20 (Affymetrix) in PBS for 60 minutes. Then, the cells were stained with appropriate primary antibodies diluted with a blocking solution at 4°C overnight. The antibodies used were rabbit anti-GFAP antibody (Abcam, diluted at 1:100), rabbit anti-S-100 antibody (Abcam, diluted at 1:100), and mouse monoclonal P0 (MBP) antibody (Abcam, diluted at 1:100). After incubation with the primary antibodies, the cells were washed three times with PBST, and incubated with Alexa-488-conjugated goat secondary anti-mouse antibody (A11001, Invitrogen) or Alexa-563-conjugated goat secondary anti-rabbit antibody (A21428, Invitrogen), diluted at 1:100, at room temperature for 2 hours. The cells were incubated with 1 µg/mL DAPI (D9542, Sigma-Aldrich) for 5 minutes at room temperature to stain the nuclei. Subsequently, the sample was visualized using a fluorescence microscope (IX71S1F3, Olympus).

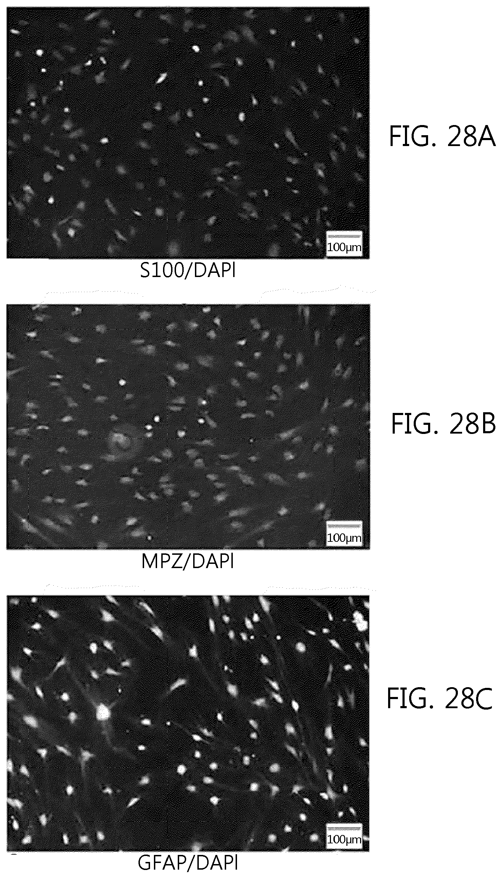

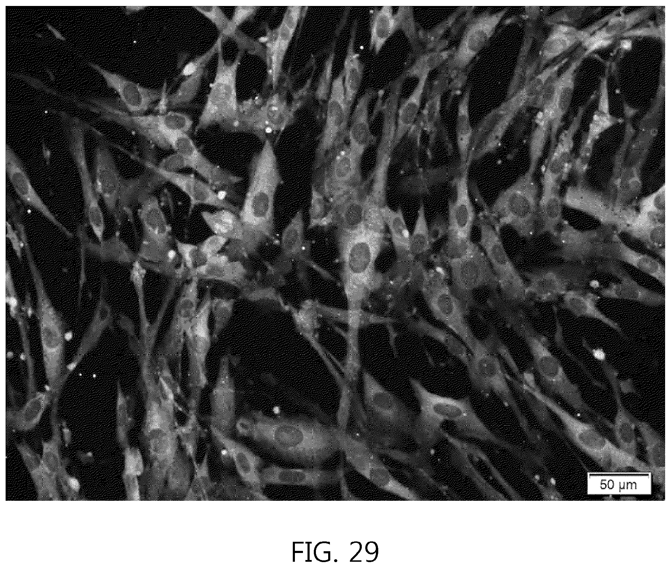

The result values for the experiment conducted in Experimental Example 3 are shown in FIGS. 28 and 29. Looking at the results in FIGS. 28 and 29, it can be confirmed that most of the glia-like cells expressed S100, MBP, and GFAP, which are marker proteins characteristic of neuroglial cells. Since the glia-like cells expressed important neuroglial cell markers essential for neurorepair and neuroprotection, it was concluded that the glia-like cells have the property of protecting and repairing neurons, similar to neuroglial cells.

### Experimental Example 4: Quantitative RT-PCR

Next, the present inventors profiled the global gene expression pattern of C1-glia-like cells at various time points and investigated for important Schwann cell/neuroglial cell markers.

Glia-like cells were harvested at various time points, total RNA was extracted therefrom using RNeasy Mini Kit (QIAGEN), and 5 µg of the total RNA was synthesized into cDNA using RNA-to-cDNA EcoDry Premix (Oligo dT, Clontech). Quantitative RT-PCR was performed using SYBR Green PCR Master Mix (Bio-Rad) on a Bio-Rad Prime PCR instrument. The qRT-PCR was performed for 40 cycles, each consisting of 95°C for 30 sec, 58°C for 15 sec, and 72°C for 15 sec. The primers used in this experiment are shown in Table 3 below.

**[Table 3] List of RT-PCR primers**

| **Primer Name** | **Forward Primer (5'- 3' )** | **Reverse Primer ( 5'- 3' )** |
|---|---|---|
| hMBP | ACTATCTCTTCCTCCCAGCTTAAAAA | TCCGACTATAAATCGGCTCACA |
| hNDRG1 | CCTTGTTGTTGTGTTGAGATCCAGT | TTTAAGCCAATCACACAAAATTCCTGG |
| hGalc | CCGAGGATACGAGTGGTGGT | TTCCCAGCCATCCAGGGAAT |
| hFBLN5 | CTCACTGTTACCATTCTGGCTC | GACTGGCGATCCAGGTCAAAG |
| hDKKl | CCTTGAACTCGGTTCTCAATTCC | CAATGGTCTGGTACITAITCCCG |
| GAPDH | TGCACCACCAACTGCTTAGC | GGCATGGACTGTGGTCATGAG |

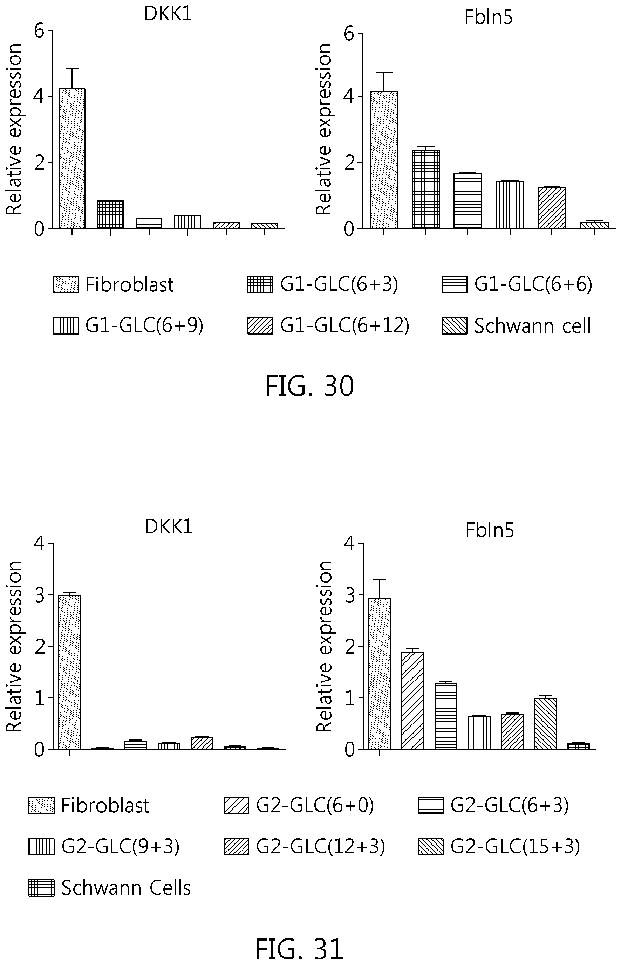

FIGS. 30 to 32 show the results of measuring the expression level of each gene by RT-PCR for somatic cells, Schwann cells, and glia-like cells produced according to the protocols of Examples 1, 2 and 3.

Looking at the results shown in FIGS. 30 to 32, it could be confirmed that the expression of DKK1 or FBLN5, which are representative marker genes of fibroblasts, was little or significantly reduced in the glia-like cells, but the expression levels of MBP, GALC and NDRG1, which are marker genes of neuroglial cells, increased in the glia-like cells. These results indicate that the glia-like cells lost the characteristics of fibroblasts and acquired the characteristics of neuroglial cells.

### Experimental Example 5: Cytokine Detection and Dot Blot

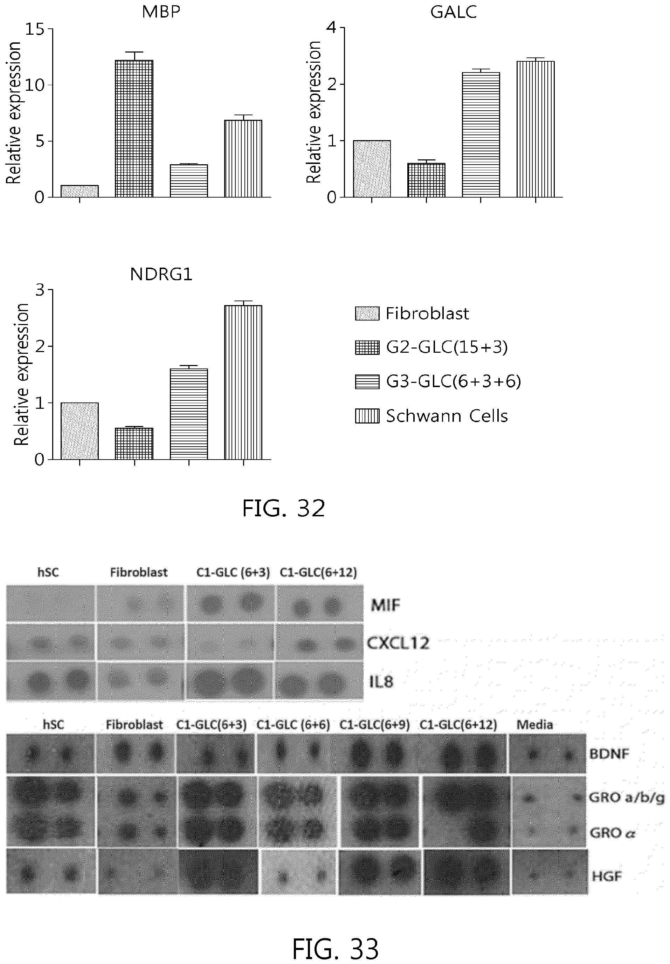

Neuroprotective and repair properties that support neuroglial cells in the peripheral and central nervous systems are generally imparted by some growth factors and cytokines released by those cells. In order to confirm whether several types of produced glia-like cells also have similar protein secretion properties, the present inventors profiled the secreted cytokines and growth factors. Fibroblasts were converted into glia-like cells (GLC) based on the protocols of Examples 1, 2 and 3. Conditioned medium was recovered in the last step of each protocol and centrifuged to remove any cell debris or particulate matter. The conditioned medium was diluted 20-fold in a dilution buffer provided in a dot blot kit (Human proteome profiler, R&D Systems). A dot blot experiment for detecting several cytokines or growth factors secreted from the conditioned media of glia-like cells produced through different protocols was performed according to the manufacturer's protocol (R&D Systems). The basal medium was used as a negative control. The secretion of cytokines or growth factors provided in the dot blot kit was confirmed through dot blots, and among these factors, factors secreted in larger amounts from the glia-like cells than from fibroblasts were those shown in FIG. 33.

Looking at the results in FIG. 33, among the several cytokines or growth factors tested, some were induced and secreted in significantly larger amounts than those from fibroblasts. For example, MIF, CXCL12, IL8, BDNF, GRO-alpha, HGF, etc. were secreted in larger amounts from the induced neuroglial cells. Among these, the amounts of MIF, BDNF and HGF secreted were significantly larger than the amounts of those from human Schwann cells (FIG. 33).

### Experimental Example 6: ELISA

Quantitation of several cytokines released into the culture medium from the glia-like cells produced according to several protocols was performed using a commercially available kit (Promega) for BDNF and GDNF and a commercially available kit (R&D Systems) for IL8, HGF, MIF and GRO-a. Fibroblasts were converted into glia-like cells according to several protocols mentioned above. As mentioned, the conditioned medium was recovered at various time times and centrifuged to remove any cell debris and particulate matter. The conditioned medium was diluted 20-fold in a dilution buffer provided in the ELISA kit. ELISA quantification was performed according to the manufacturer's protocol. The basal medium was used as a negative control. In this experiment, while 3.2 × 10⁶ cells were grown for 3 days in a 60-mm culture dish having an area of 28.2 cm², the concentration of each factor secreted into the culture medium was measured.

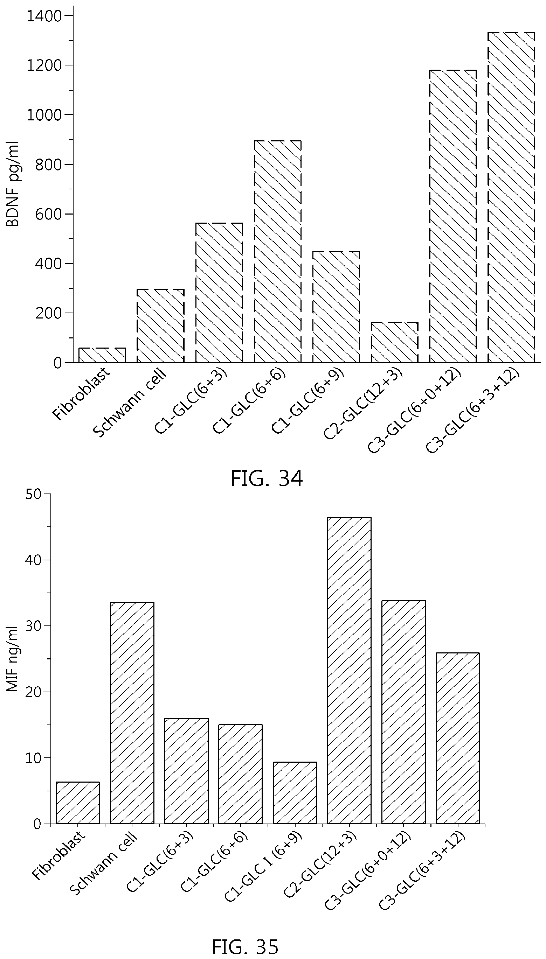

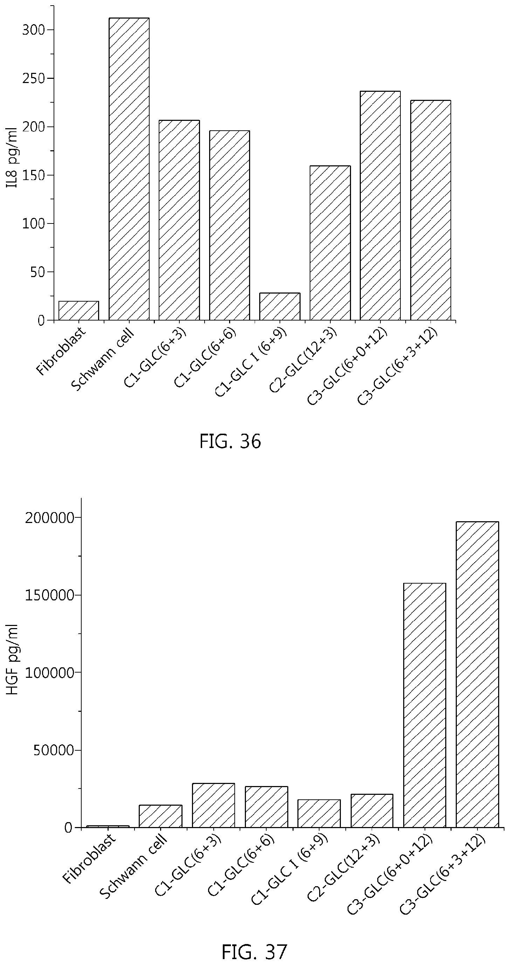

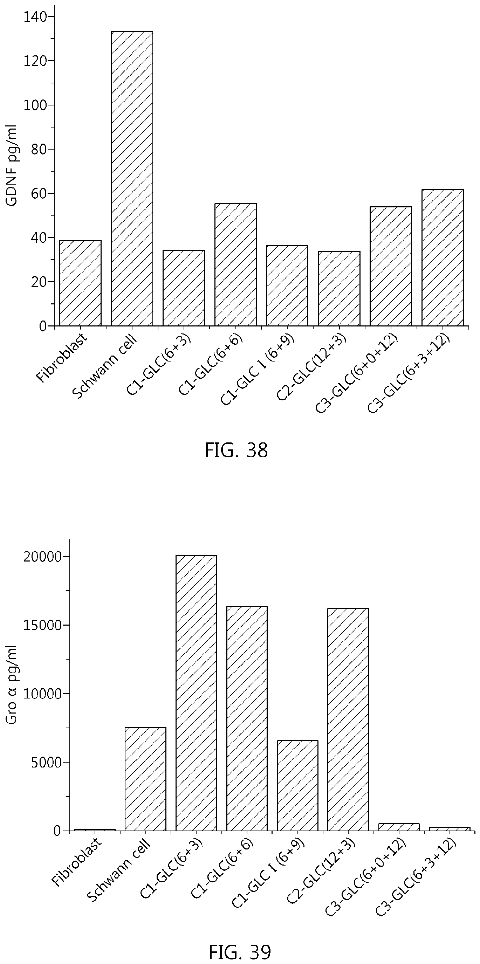

The result values for Experimental Example 6 are described in FIGS. 34 to 39.

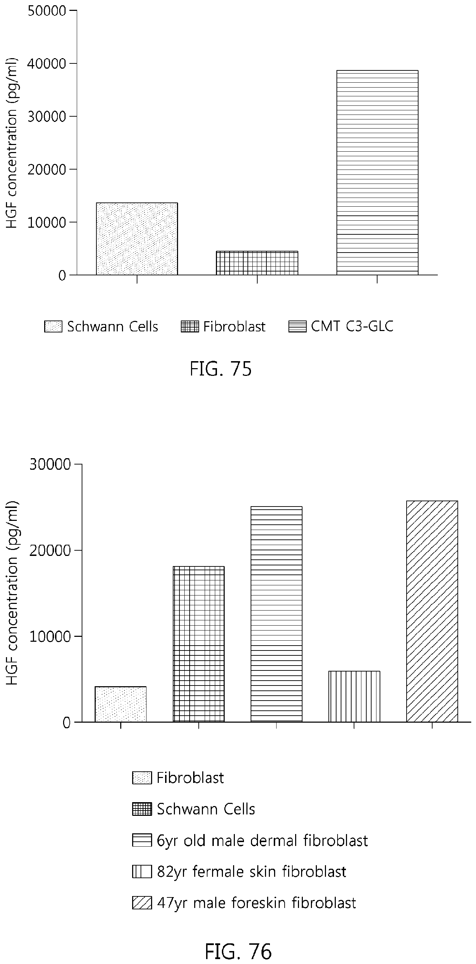

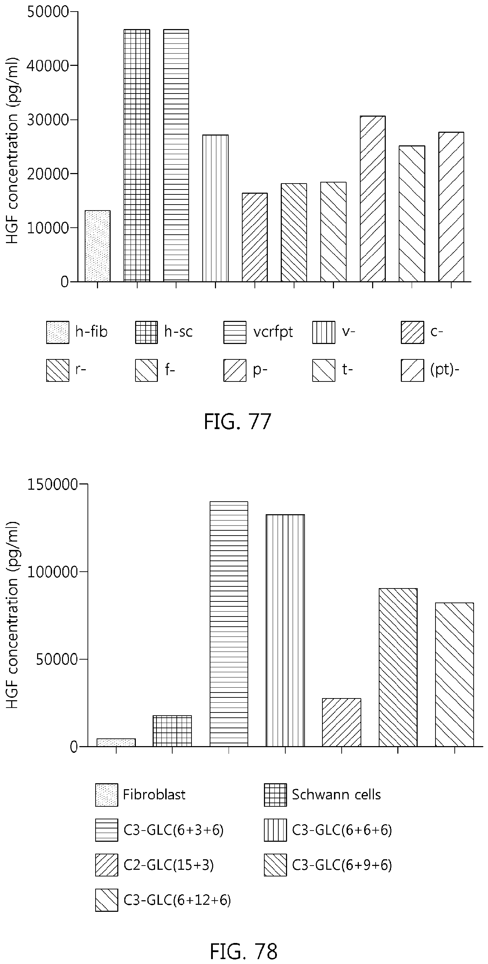

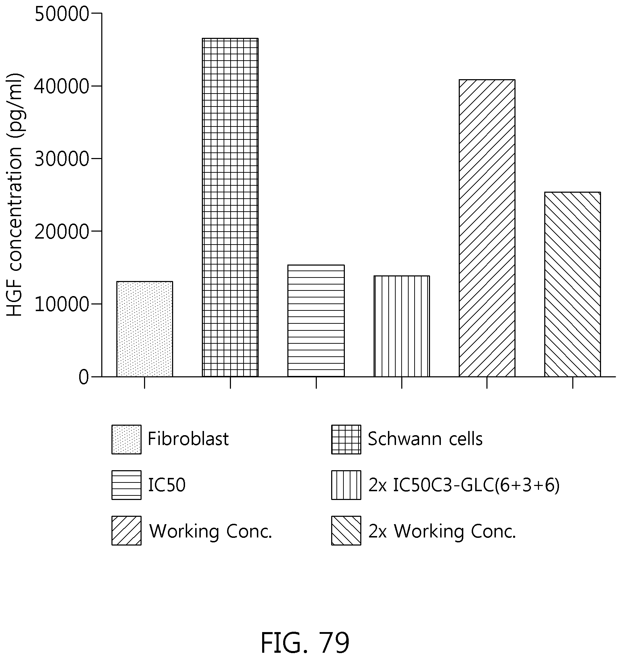

Looking at the results in FIGS. 34 to 39, it was confirmed that the secretion of most cytokines or growth factors was significantly higher in glia-like cells (GLC) that are differentiated from somatic cells than in fibroblasts (see FIGS. 34 to 39). Interestingly, C3-glia-like cells (GLC) produced according to protocol III of Example 3 secreted BDNF, GDNF, MIF, IL8 and HGF in larger amounts than glia-like cells (C1-GLC and C2-GLC) produced in other Examples (see FIGS. 34 to 39), and C3-glia-like cells secreted significantly more HGF than human Schwann cells and the glia-like cells produced according to other protocols (see FIGS. 75 to 79).

### Experimental Example 7: Preparation of Conditioned Medium for Glia-Like Cells and Analysis of Neurite Outgrowth Promotion

Schwann cells are known to secrete many neurotrophic and neuroprotective factors that promote the health and functionality of axons and aid in axonal regeneration. In order to confirm whether the chemically induced glia-like cells of the present invention belonging to several classes correspond to functional physiological counterparts, the present inventors prepared a conditioned medium, treated NSC-34 motor neurons with the conditioned medium, and evaluated the effect of the conditioned medium on neurite outgrowth.

According to the protocols shown in Examples 1 to 3, fibroblasts were converted into glia-like cells (GLC). In each case, at the end of the conversion, the conditioned medium was recovered and centrifuged to remove any cell debris or particulate matter. This conditioned medium was stored at -80°C for later use.

Analysis of neurite outgrowth promotion was performed using NSC-34 motor neuron cells. These cells were kept in high-glucose DMEM (Welgene) supplemented with 10% FBS and 1% penicillin and streptomycin (Welgene). For analysis, these cells were washed with 1× PBS, transferred to a conditioned medium for SC cells, and then grown for an additional 48 hours. Next, neurite promotion was evaluated by microscopic analysis and quantified using ImageJ.

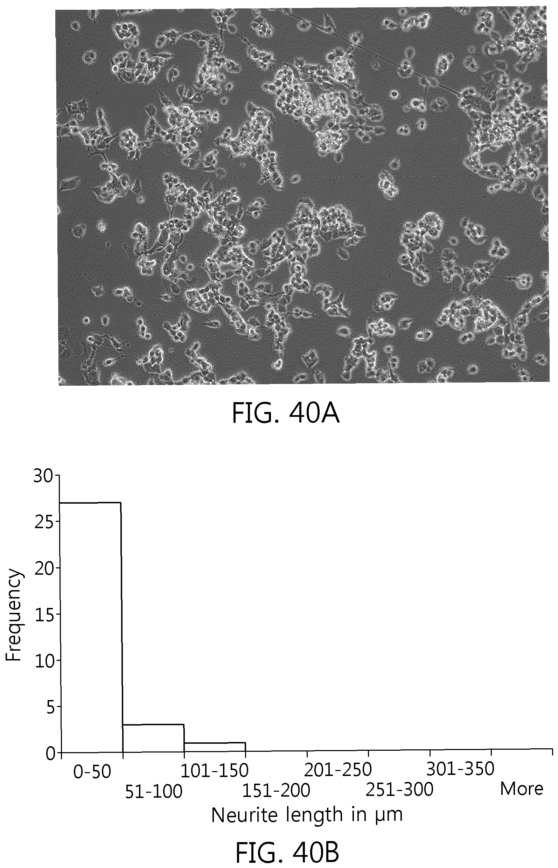

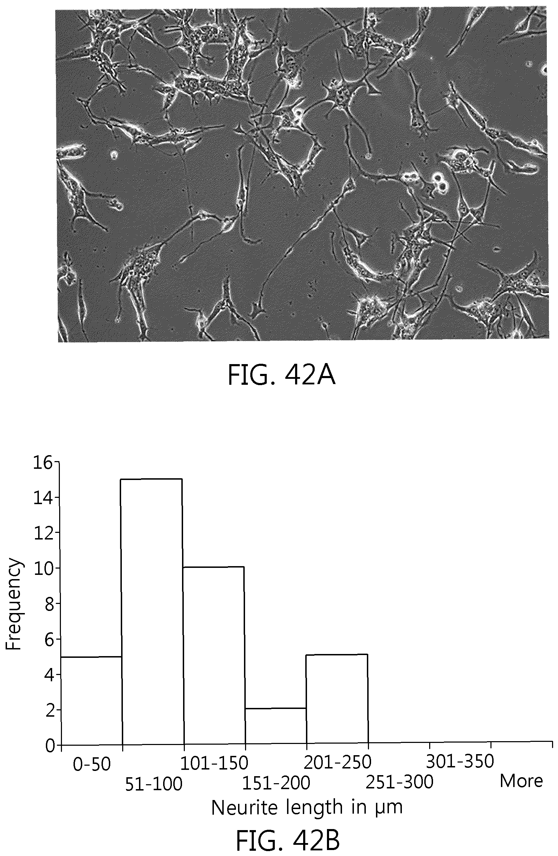

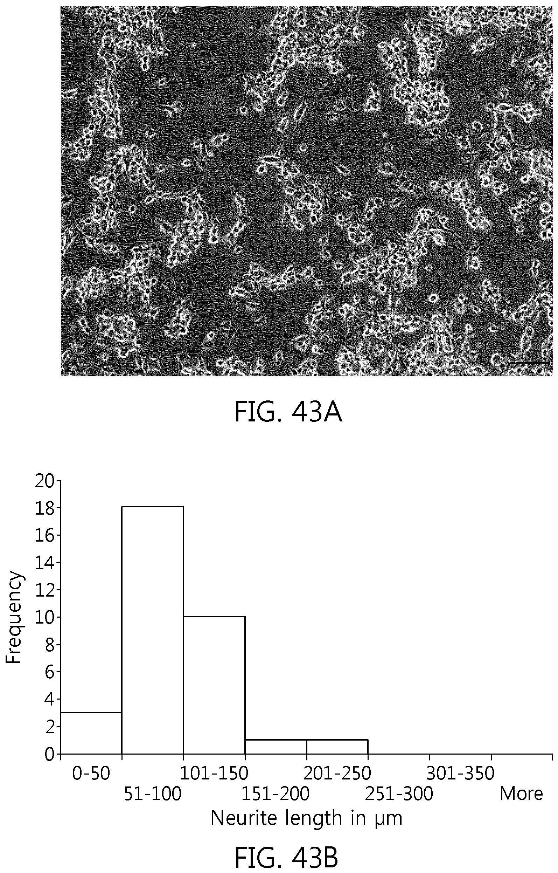

The result values are shown in FIGS. 40 to 44.

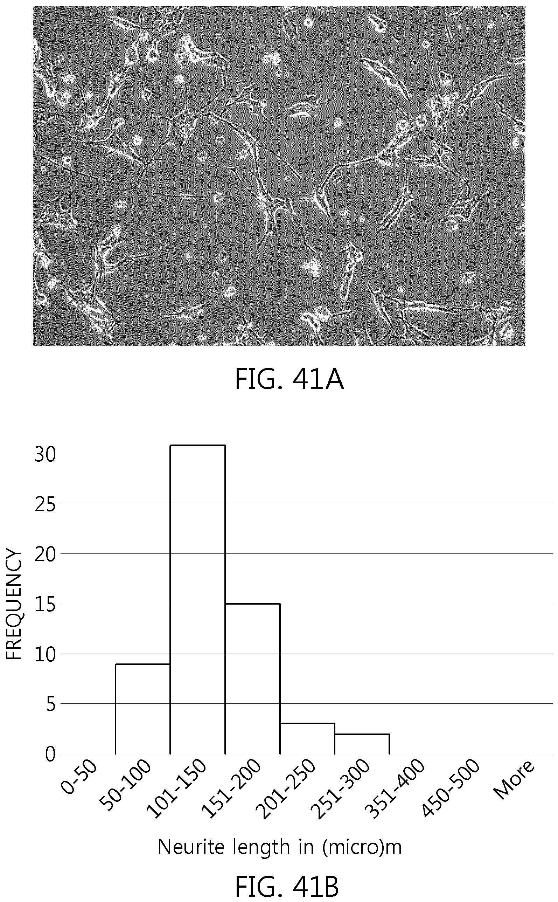

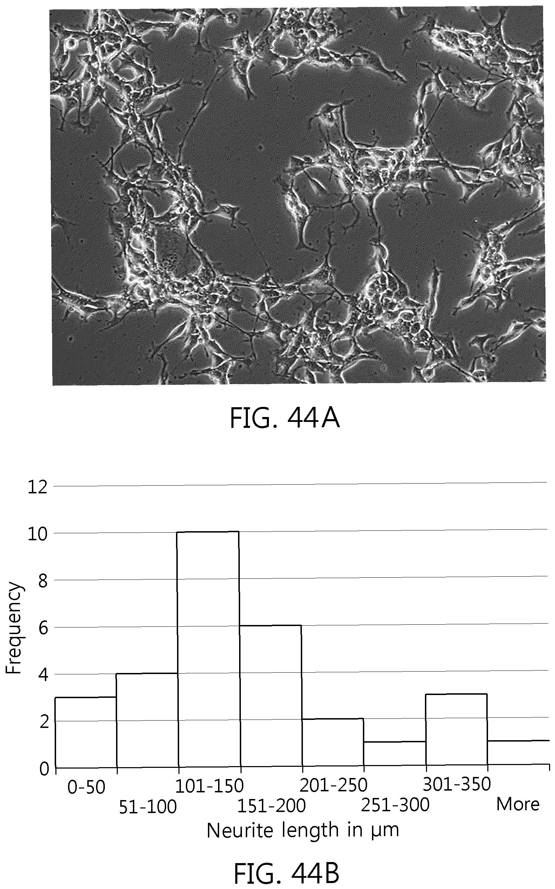

Looking at the results in FIGS. 40 to 44, consistent with our hypothesis, the conditioned medium promoted neurite outgrowth (FIGS. 41, 42 and 43A), very similar to the conditioned medium for Schwann cells (FIG. 44A). However, this growth promoting activity was not found in the conditioned medium for fibroblasts (FIG. 40A). In addition, as a result of quantifying the degree of neurite outgrowth caused by the conditioned medium, it could be confirmed that, when motor neurons were treated with the glia-like cell conditioned medium, the number of motor neurons having long neurites increased, like when motor neurons were treated with the Schwann cell conditioned medium (FIGS. 41, 42 and 43B). However, when motor neurons were treated with the fibroblast conditioned medium, neurons having long neurites were hardly found (FIG. 40B).

### Experimental Example 8: Differentiation Efficiency

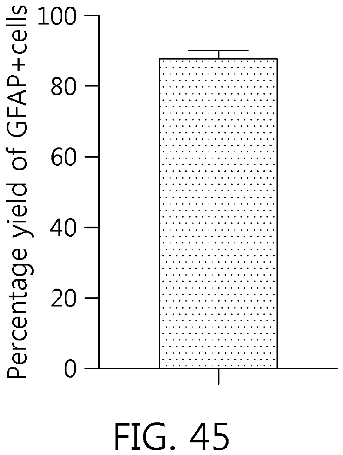

Glia-like cells were immunostained with GFAP antibody, a characteristic marker of neuroglial cells, and quantification of conversion efficiency was performed by counting immunopositive cells based on DAPI-stained nuclei (FIG. 28). The yield of conversion to glia-like cells was 85% as determined by cells showing the expression of GFAP (FIG. 45).

### Experimental Example 9: Proliferation Ability of C3-Glia-Like Cells (5-ethynyl-2'-deoxyuridine staining assay)

One of the important features with regard to the applicability of glia-like cells in the field of regenerative medicine and therapy is the ability of cell proliferation and expansion. In order to evaluate these features, on day 6 of reculture, Edu was incorporated into C3-glia-like cells for 12 hours, and then cell preparation was performed using Click-iT™ EdU Alexa Fluor™ 555 (Invitrogen, MA, USA) according to the manufacturer's protocol. Next, the cells were co-stained with Hoechst33342 to detect nuclei. EDU + Hoechst33342 positive cells were counted in two separate microscopic fields and expressed as the percentage of total Hoechst33342 positive cells present in the microscopic fields.

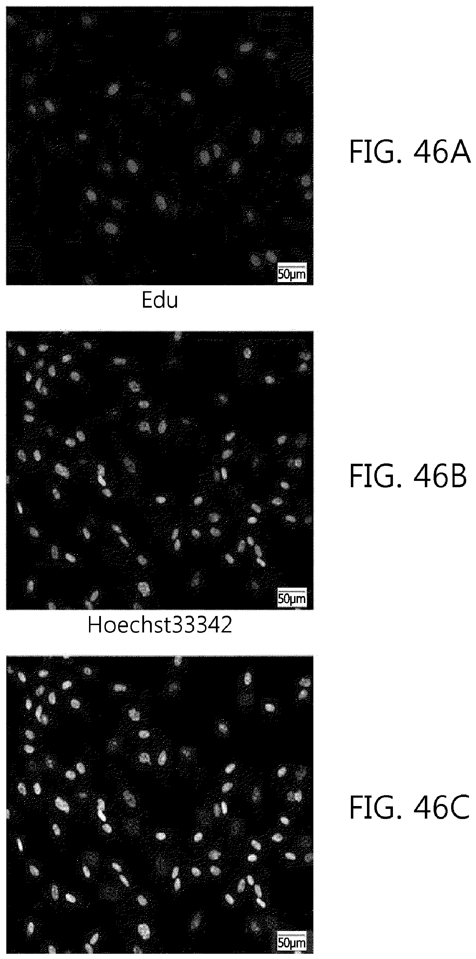

The result values are shown in FIGS. 46 and 47.

Looking at the results in FIGS. 46 and 47, in fact, the immunofluorescence data indicates that C3-glia-like cells had mitotic activity (FIG. 46), and the quantitative results indicate that 30% of the cells in the culture medium were Edu/DAPI double-positive cells (FIG. 47).

### Experimental Example 10: Freezing and Thawing of Glia-Like Cells

One of the most important points in the development of therapeutic agents using glia-like cells is that the produced cells should be capable of being stably stored and transported. One of the best ways to store cells is to freeze the cells at a low temperature. In order to investigate the change in activity when freezing and thawing the glia-like cells produced through the present technology, the cells were cultured in a C3-G1C freezing medium (high-glucose DMEM containing 20% FBS and 10% DMS), and then frozen rapidly in liquid nitrogen rapidly. After one week, the cells were thawed and recultured in Schwann cell culture medium, and the characteristics of the cells were examined during the reculture.

The result values are shown in FIGS. 48 to 51.

Looking at the results in FIGS. 48 to 51, it could be confirmed that the cells thawed after freezing maintained the same cell morphology as the cells before freezing (FIG. 48), and when the thawed cells were recultured, they secreted a larger amount of HGF than fibroblasts and human Schwann cells, even though the amount of HGF secreted slightly decreased compared to the amount of HGF secreted from the cells before freezing (FIG. 49). In addition, it could be confirmed that, when neurite outgrowth of motor neurons was induced using the thawed cell culture medium, the culture medium showed activity similar to the cells before freezing (FIGS. 50 and 51). These results can confirm that the present disclosure may provide glia-like cells that can be used even after long-term storage.

### Experimental Example 11: Establishment of Rat CCI Model and Transplantation of Glia-Like Cells

In order to confirm the applicability of glia-like cells that are differentiated from somatic cells as a cell therapy product, it is necessary to confirm the neuroprotective and neuroregenerative functions of these cells in an animal model. To this end, a rat chronic constriction injury (CCI) model was created by injuring the rat femoral nerve, and human fibroblasts, human Schwann cells and glia-like cells were transplanted into the injured nerve. 8 weeks after transplantation, the degree of nerve regeneration was analyzed by measuring the degree of nerve regeneration through various methods.

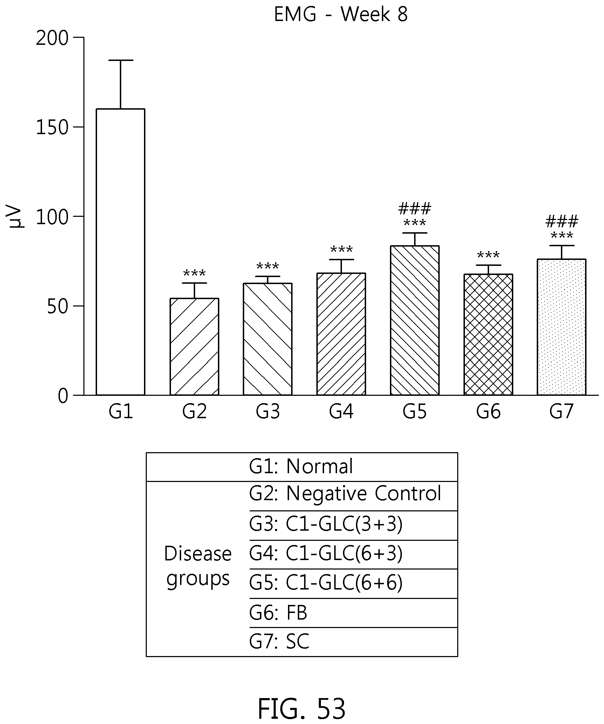

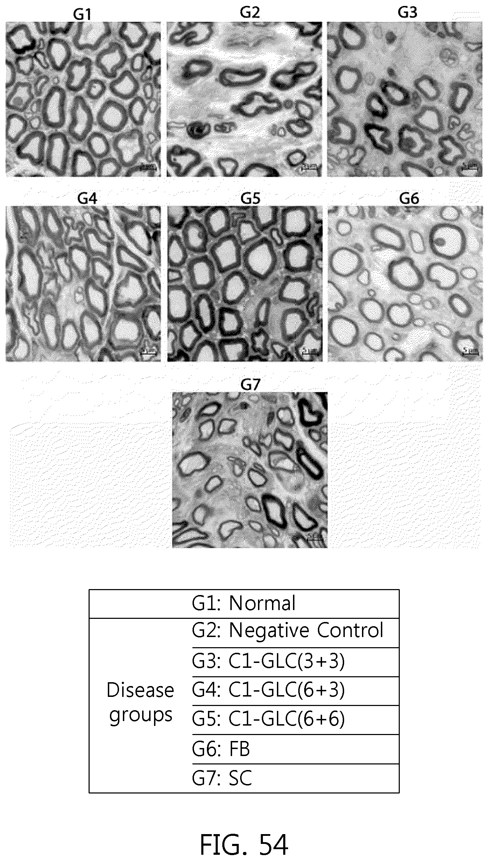

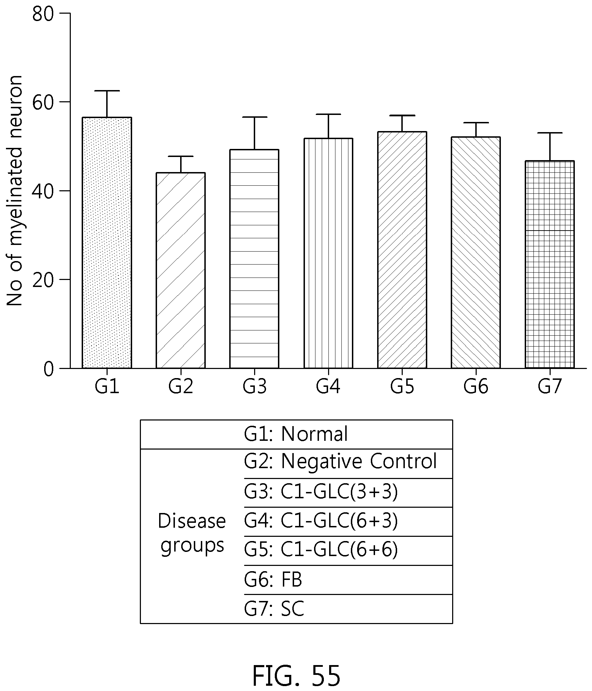

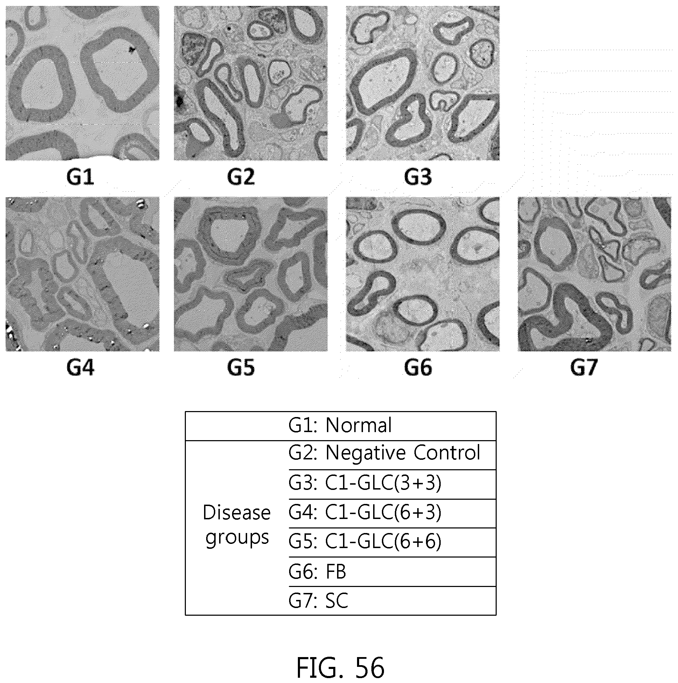

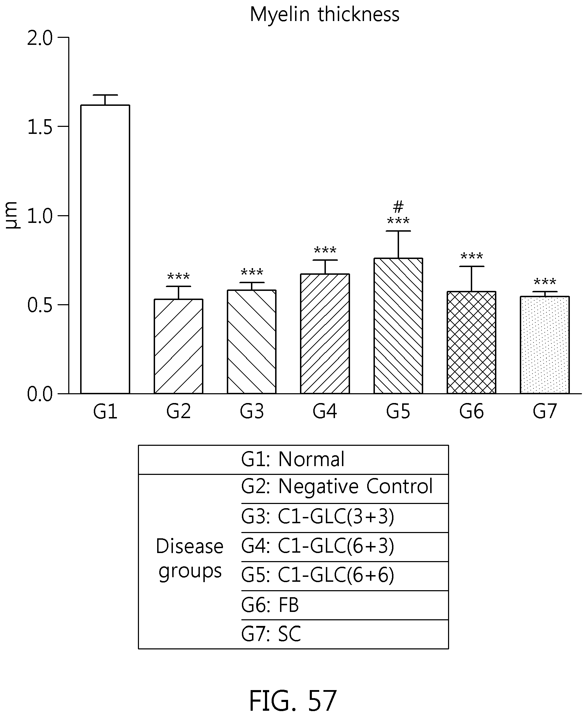

A rat CCI model was prepared by exposing the sciatic nerve through surgery and then constricting the nerve with a surgical suture. At the end of maturation, various types of glia-like cells produced according to various protocols were harvested, and 5.5×10⁵ cells in PBS were mixed with the same amount of Tissel solution and transplanted into each rat. After 8 weeks, therapeutic improvement was evaluated by a rotarod latency test, electromyography, and staining assay, and compared with that in rats without transplantation or wild-type rats without nerve injury. Human Schwann cells and fibroblasts were also tested in the same manner. The results of the rotarod test (FIG. 52) and electromyography (EMG) (FIG. 53) prove that the glia-like cells (G3 to G5) have a neurorepair function, unlike control groups (G2, untreated; G6, treated with fibroblasts; G7, treated with human Schwann cells). In addition, the results of quantifying the number of neurons restored by myelin using a staining photograph (FIG. 54) of the sciatic nerve (FIG. 55) also indicated that the test group showed a high therapeutic effect compared to the control groups. In addition, as a result of observing the axon part of the sciatic nerve with an electron microscope (FIG. 56) and observing the thickness of the myelin layer (FIG. 57), it could be confirmed that the myelin layer was significantly thicker in the test group than in the control groups (FIG. 57). These results confirm that the glia-like cells induced using the small molecular compounds promoted neuroregeneration in the rat CCI model.

### Experimental Example 12: Examination of Concentration-Dependent Effect of Cocktail Compounds

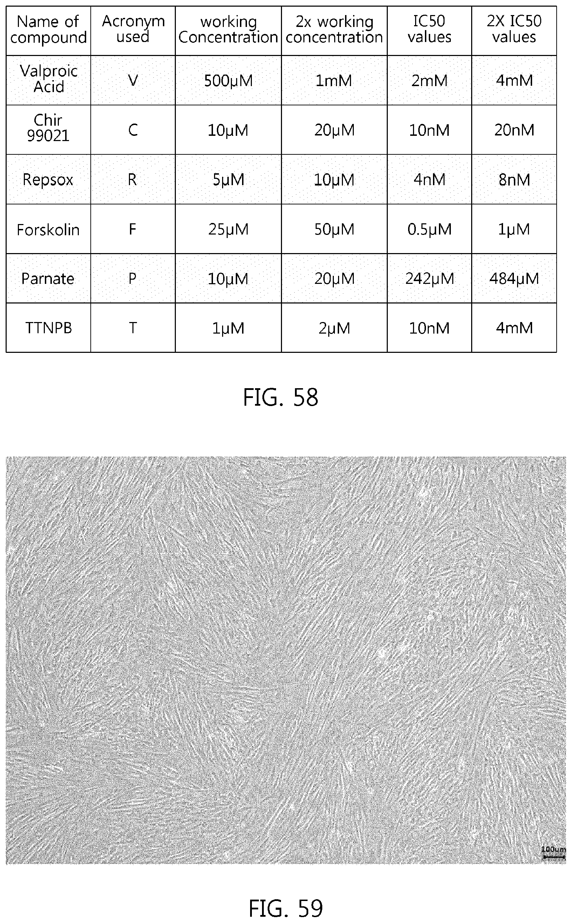

The doses of chemicals used in the cocktail required for conversion into glia-like cells were selected from the IC₅₀ values of the chemicals used and the doses reported in the literature. For further optimization and to get an idea of the dose ranges of the chemicals used for conversion, the present inventors selected several dose ranges, such as 2x working concentrations and 2x IC₅₀ values, and conducted a cell conversion experiment using the selected dose ranges. The present inventors monitored the conversion yield along with the toxicities of the chemicals used. Chemical cocktails were prepared as shown in Table 4 below (FIG. 58), and the results of evaluating the effects thereof on differentiation are shown in FIGS.

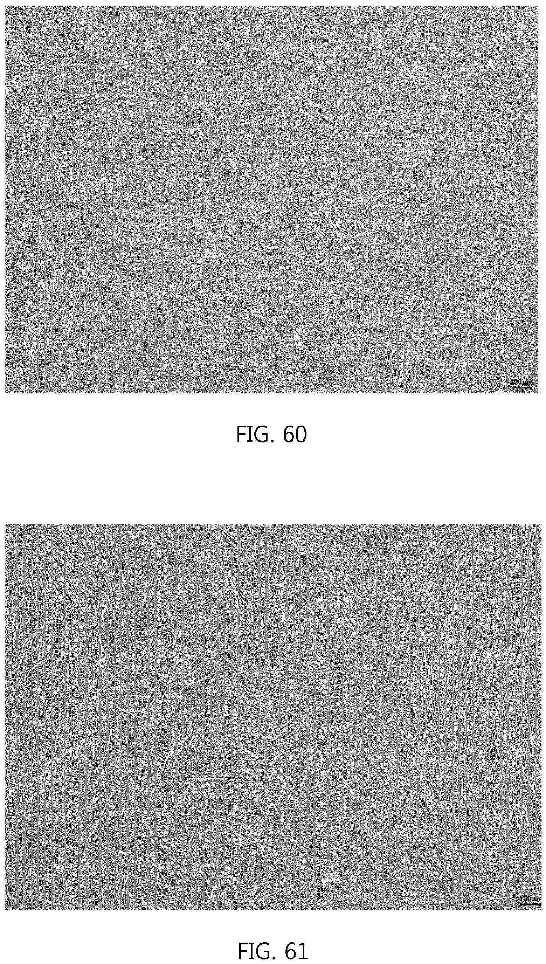

59 to 62. Looking at the results in FIGS. 59 to 62, it could be confirmed that, when concentrations greater than the optimized concentrations were used, some toxicity to the cells appeared, but cell conversion was effectively achieved. In addition, it could be confirmed that, when IC₅₀ values or approximately 2x IC₅₀ values were used, cell conversion was not effectively achieved.

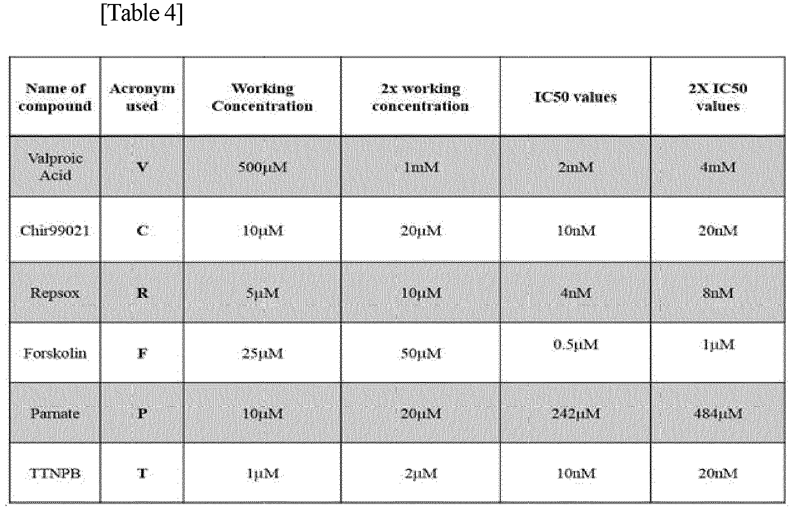

### Experimental Example 13: Differentiation into Glia-Like Cells Using Various Types of Somatic Cells

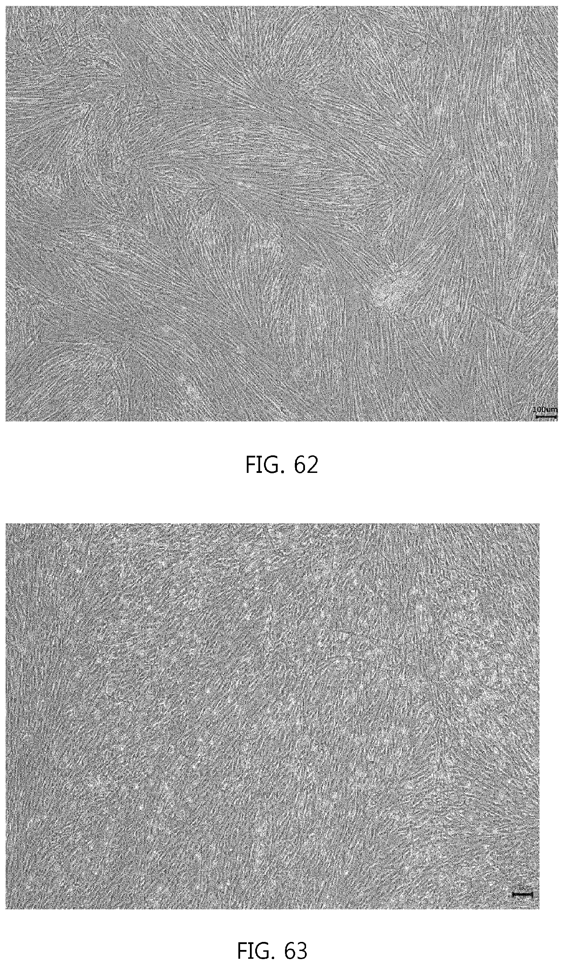

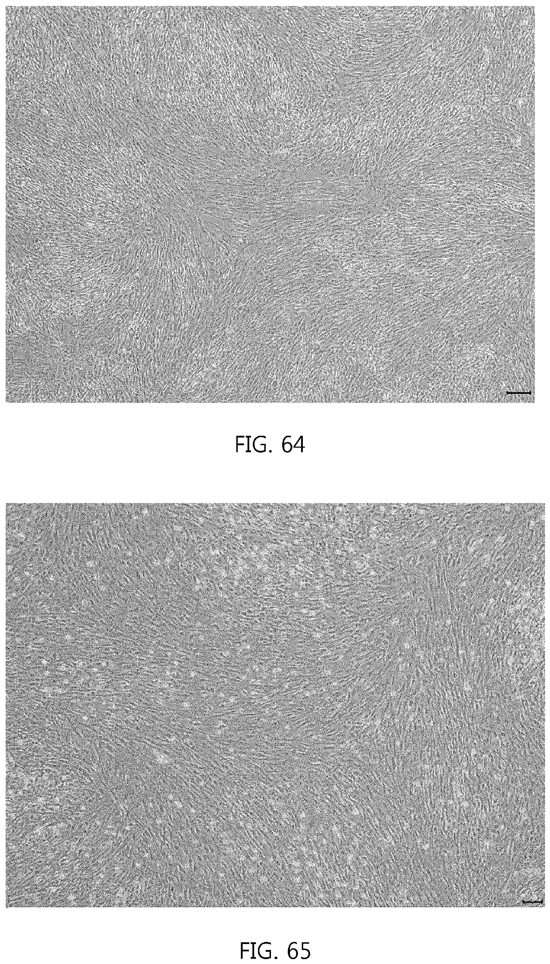

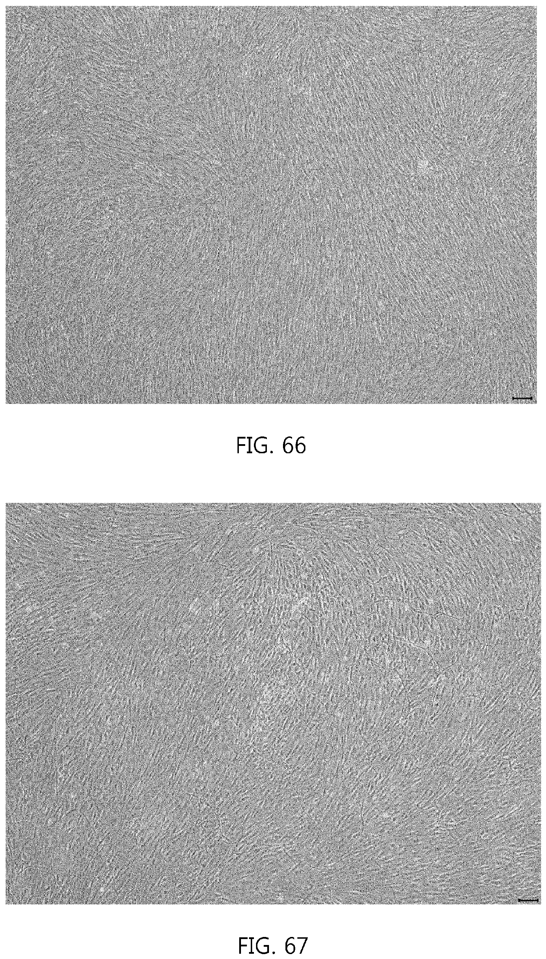

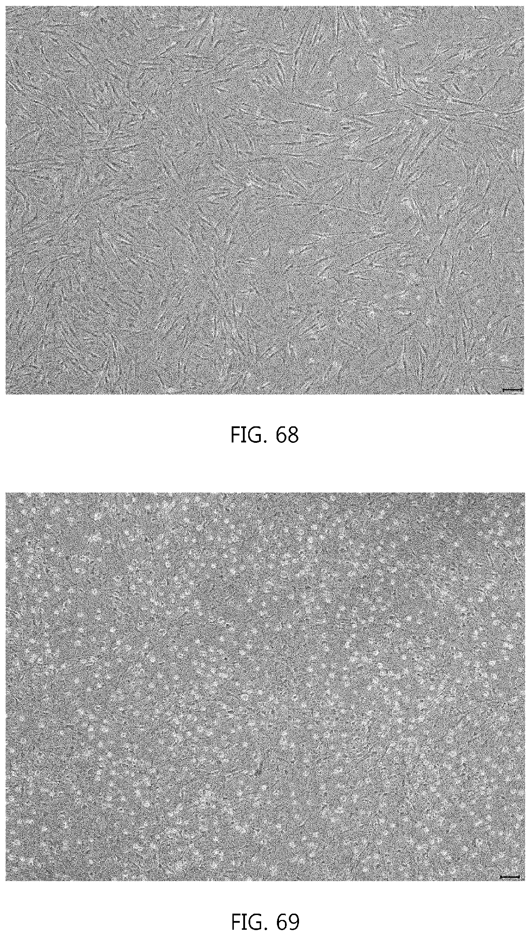

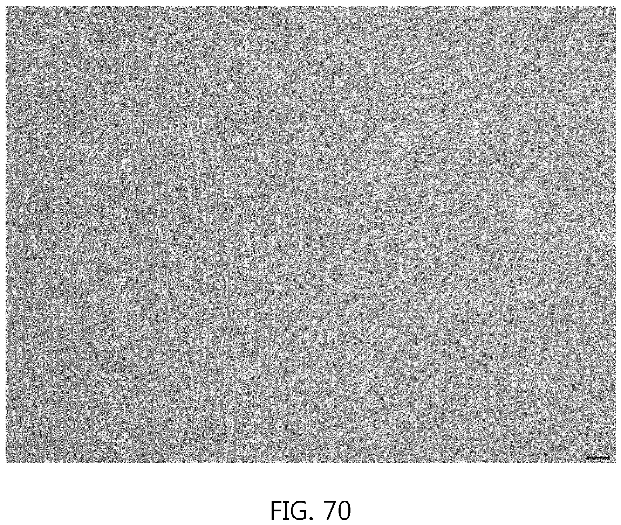

In order to use the present technology for the development of patient-specific cell therapy products, it should be confirmed that the present technology is applicable to various types of fibroblasts. Thus, C2-GLC (15+3) and C3-GLC (6+3+6) were produced according to the same methods as the methods of Examples 2 and 3, except that fibroblasts derived from the skin of a patient suffering from CMT (Charcot-Marie-Tooth) disease and three different fibroblasts purchased from Coriell Institute were used. Differentiation into glia-like cells was determined based on the morphology of the differentiated cells through microscopic observation.
(1) Fibroblasts derived from the skin of a patient suffering from CMT (Charcot-Marie-Tooth) disease (FIGS. 63 and 64)
(2) Fibroblasts derived from the dermis of a 6-year-old boy (FIGS. 65 and 66)
(3) Fibroblasts derived from the skin of a 823-year-old female (FIGS. 67 and 68)
(4) Fibroblasts derived from the foreskin of a 47-year-old male (FIGS. 69 and 70)

The results of differentiation of the cells into glia-like cells are shown in FIGS. 63 to 70.

The results of this experiment could confirm that, even when somatic cells of various origins including patients are used, conversion from these cells to glia-like cells is possible.

In addition, looking at the results in FIGS. 75 and 76, it could be confirmed that a large amount of HGF was secreted even from glia-like cells that are differentiated from fibroblasts of various human origins including patients.

Looking at the results in FIGS. 63 to 70 and 75 to 76, it can be seen that various fibroblasts of human origin could be effectively differentiated into glia-like cells. These results mean that, when fibroblasts from a patient are used in the future development of a cell therapy product containing glia-like cells, it is possible to develop a cell therapy product that does not have side effects such as immune rejection, which becomes a problem in cell therapy products obtained or derived from other persons.

### Experimental Example 14: Establishment of Rat SCI Model and Transplantation of Glia-Like Cells

In order to confirm the applicability of glia-like cells that are differentiated from somatic cells as a cell therapy product for central nervous system-related diseases, it is necessary to confirm the neuroprotective and neuroregenerative functions of these cells in an animal model. To this end, a rat spinal cord injury (SCI) model was created by injuring the rat spinal cord, and human Schwann cells and glia-like cells were transplanted into the injured nerve. 2 weeks after transplantation, the degree of nerve regeneration was analyzed by measuring the degree of nerve regeneration through various methods.

A rat SCI model was prepared by exposing the spinal cord through surgery and then applying pressure thereto. At the end of maturation, various types of glia-like cells produced according to various protocols were harvested, and 5.5×10⁵ cells in PBS were transplanted into the rat spinal cord. After 2 weeks, therapeutic improvement was evaluated by a rotarod latency test and BBB scoring, and compared with that in rats without transplantation or wild-type rats without nerve injury. Human Schwann cells were also tested in the same manner. Looking at the results of the rotarod test and BBB scoring (FIG. 80), it could be seen that, immediately after injury (week 0), the activity was markedly reduced in all the injured groups, but 2 weeks after cell transplantation, the group (G3) treated with the glia-like cells and the group (G4) treated with the human Schwann cells showed a neurorepair function, unlike and the control group (G2, untreated). In addition, it could be observed that the therapeutic effect in the group (G3) treated with the glia-like cells was more improved than that in the group (G4) treated with the human Schwann cells. These results confirm that the glia-like cells induced using the small molecular compounds promote neuroregeneration in the rat SCI model. In addition, these results indicate that the glia-like cells can be used as a cell therapy product for central nervous system diseases.

### Experimental Example 15: In Vivo Residence After Transplantation of Glia-Like Cells

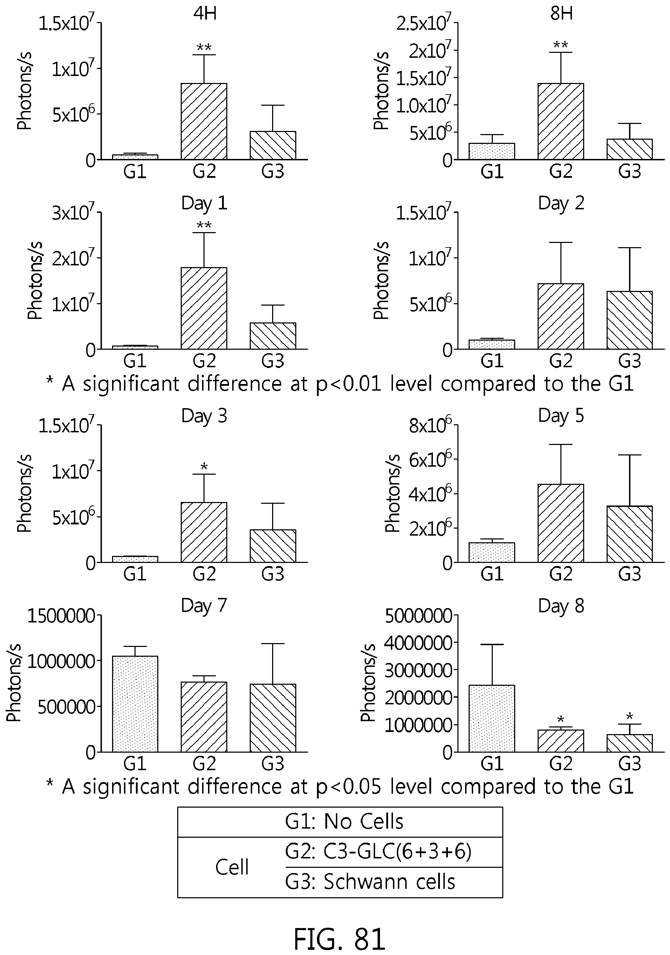

For the applicability of the glia-like cells that are differentiated from somatic cells as a cell therapy product, it is necessary to confirm how long the cells stay *in vivo* after transplantation *in vivo.* Using a virus, the gene of green fluorescence protein (GFP) was expressed in glia-like cells or human Schwann cells, and 5.5×10⁵ GFP-expressing glia-like cells or human Schwann cells in PBS were mixed with the same amount of Tissel solution and transplanted into the rat's thigh. After a predetermined time point, the intensity of fluorescence generated in the rat's thigh was measured through fluorescence imaging (FIG. 81).

When analyzing the results shown in FIG. 81, fluorescence could be observed during a period from immediately after cell injection to day 5, but no fluorescence could be observed from day 7. This result suggests that the injected cells are dead between day 5 and day 6.

This result confirmed that, even when human cells were injected into wild-type rats without immunocompromising, the transplanted cells could stay *in vivo* for at least 5 days. Therefore, it is concluded that, when glia-like cells produced using cells isolated from a living body or patient without immune activity are transplanted into the patient, the transplanted cells may exhibit activity for a longer period of time.

### Industrial Applicability

The present disclosure provides novel glia-like cells that are differentiated from somatic cells, a method for producing the same, a cell therapy product for treating neurological disorder containing the same, and a method of preventing and treating neurological disorder by administering the above-described cells.

### Prior Art Documents

### Patent Documents

Patent Document 1: Korean Patent Application Publication No. 10-2017-0045356

## Claims

1. Glia-like cells that are differentiated from somatic cells and secrete 20,000 pg/ml or more of HGF.

2. The glia-like cells of claim 1, which further secrete 150 pg/ml or more of BNDF and 10 ng/ml or more of MIF

3. The glia-like cells of claim 1, wherein the glia-like cells are produced by a method comprising a differentiation induction step of inducing differentiation of the somatic cells by treatment with a first chemical cocktail containing a histone deacetylase inhibitor, a GSK inhibitor, an ALK-5 kinase inhibitor, a cAMP agonist, and a histone demethylase inhibitor.

4. The glia-like cells of claim 3, wherein the glia-like cells are produced by the method further comprising a re-culture step after the differentiation induction step.

5. The glia-like cells of claim 3, wherein the first chemical cocktail further contains an RAR agonist.

6. The glia-like cells of claim 1, wherein the somatic cells are at least one type selected from the group consisting of fibroblasts; fully differentiated somatic cells, including blood cells and adipocytes; and adult stem cells present in umbilical cord, placenta, umbilical cord blood, bone marrow, blood, and fat.

7. The glia-like cells of claim 1, wherein the somatic cells are skin-derived fibroblasts.

8. The glia-like cells of claim 7, wherein the skin is at least one selected from the group consisting of epidermis, dermis and fat layers.

9. The glia-like cells of claim 7, wherein the skin-derived fibroblasts are foreskin-derived fibroblasts.

10. Glia-like cells that are differentiated from somatic cells, the glia-like cells being produced by a method comprising a differentiation induction step of inducing differentiation of the somatic cells by treatment with a first chemical cocktail containing a histone deacetylase inhibitor, a GSK inhibitor, an ALK-5 kinase inhibitor, a cAMP agonist, and a histone demethylase inhibitor.

11. The glia-like cells of claim 10, wherein the glia-like cells secrete 20,000 pg/ml or more of HGF.

12. The glia-like cells of claim 11, which further secrete 150 pg/ml or more of BNDF and 10 ng/ml or more of MIF

13. A method for producing glia-like cells that are differentiated from somatic cells, the method comprising a differentiation induction step of inducing differentiation of the somatic cells by treatment with a first chemical cocktail containing a histone deacetylase inhibitor, a GSK inhibitor, an ALK-5 kinase inhibitor, a cAMP agonist, and a histone demethylase inhibitor.

14. The method of claim 13, further comprising a re-culture step after the differentiation induction step.

15. The method of claim 14, wherein the re-culture step comprises a step additionally culturing the glia-like cells that are differentiated from somatic cells in a culture medium free of low-molecular compounds and Matrigel.

16. The method of claim 13, further comprising a step of maturing the somatic cells, treated with the first chemical cocktail, by further treatment with a second chemical cocktail containing a GSK inhibitor, an ALK-5 kinase inhibitor, and a cAMP agonist.

17. The method of claim 13, wherein the differentiation induction step is performed for at least 3 days.

18. The method of claim 15, wherein the re-culture step is performed for at least 3 days.

19. The method of claim 13, wherein the first chemical cocktail further contains an RAR agonist.

20. A cell therapy product for treating neurological disorder containing, as an active ingredient, the glia-like cells that are differentiated from somatic cells according to any one of claims 1 to 12.

21. A chemical cocktail composition for producing glia-like cells that are differentiated from somatic cells, the chemical cocktail composition containing a histone deacetylase inhibitor, a GSK inhibitor, an ALK-5 kinase inhibitor, a cAMP agonist and a histone demethylase inhibitor.

22. The chemical cocktail composition of claim 21, further containing an RAR agonist.

23. The chemical cocktail composition of claim 21, wherein the histone deacetylase inhibitor is at least one selected from the group consisting of valproic acid, pracinostat, trichostatin A, 2,4-pyridinedicarboxylic acid, suberoylanilide hydroxamic acid, hydroxamic acid, cyclic tetrapeptide, depsipeptides, vorinostat, belinostat, panobinostat, benzamide, entinostat, and butyrate.

24. The chemical cocktail composition of claim 21, wherein the GSK inhibitor is at least one selected from the group consisting of Chir99021 (6-(2-(4-(2,4-dichlorophenyl)-5-(4-methyl-1H-imidazol-2-yl)pyrimidin-2-ylamino)ethylamino) nicotinonitrile); LY2090314 (3-imidazo[1,2-a]pyridin-3-yl4-[1,2,3,4-tetrahydro-2-(1-piperidinylcarbonyl)-pyrrolo[3,2jk][1,4] benzodiazepin-7-yl]); 1-azakenpaullone (9-bromo-7,12-dihydro-pyrido[3',2':2,3]azepino[4,5-b]indol-6(5H)-one); BIO ((2'Z,3'E)-6-bromoindirubin-3'-oxime); ARA014418 (N-(4-methoxybenzyl)-N'-(5-nitro-1,3-thiazol-2-yl)urea); indirubin-3'-monoxime; 5-iodo-indirubin-3'-monoxime; kenpaullone (9-bromo-7,12-dihydroindolo-[3,2-d][1]benzazepin-6(5H)-one); SB-415286 (3-[(3-chloro-4-hydroxyphenyl)amino]-4-(2-nitro-phenyl)-1H-pyrrole-2,5-dione); SB-216763 (3-(2,4-dichlorophenyl)-4-(1-methyl-1H-indol-3-yl)-1H-pyrrole-2,5-dione); Maybridge SEW00923SC (2-anilino-5-phenyl-1,3,4-oxadiazole); (Z)-5-(2,3-methylenedioxyphenyl)-imidazolidine-2,4-dione; TWS 119 (3-(6-(3-aminophenyl)-7H-pyrrolo[2,3-d]pyrimidin-4-yloxy)phenol); Chir98014 (N2-(2-(4-(2,4-dichlorophenyl)-5-(1H-imidazol-1-yl)pyrimidin-2-ylamino)ethyl)-5-nitropyridine-2,6-diamine); SB415286 (3-(3-chloro4-hydroxyphenylatnino)-4-(2-nitrophenyl)-1H-pyrrole-2,5-dione); and Tideglusib (2-(1-naphthalenyl)-4-(phenylmethyl)).

25. The chemical cocktail composition of claim 21, wherein the ALK-5 kinase inhibitor is at least one selected from the group consisting of RepSox (1,5-naphthyridine, 2-[3-(6-methyl-2-pyridinyl)-1H-pyrazol-4-yl]); SB431542 (4-(4-(benzo[d][1,3]dioxol-5-yl)-5-(pyridin-2-yl)-1H-imidazol-2-yl)benzamide); SB525334 (6-(2-tert-butyl-4-(6-methylpyridin-2-yl)-1H-imidazol-5-yl)quinoxaline); GW788388 (4-(4-(3-(pyridin-2-yl)-1H-pyrazol-4-yl)pyridin-2-yl)-N-(tetrahydro-2H-pyran-4-yl)benzamide); SD-208 (2-(5-chloro-2-fluorophenyl)-N-(pyridin-4-yl)pteridin-4-amine); Galunisertib (LY2157299, 4-(2-(6-methylpyridin-2-yl)-5,6-dihydro-4H-pyrrolo[1,2-b]pyrazol-3-yl)quinoline-6-carboxamide); EW-7197 (N-(2-fluorophenyl)-5-(6-methyl-2-pyridinyl)4-[1,2,4]triazolo[1,5-a]pyridin-6-yl-1H-imidazole-2-methanamine); LY2109761 (7-(2-morpholinoethoxy)-4-(2-(pyridin-2-yl)-5,6-dihydro-4H-pyrrolo[1,2-b]pyrazol-3-yl)quinoline); SB505124 (2-(4-(benzo[d][1,3]dioxol-5-yl)-2-tert-butyl-1H-imidazol-5-yl)-6-methylpyridine); LY364947 (quinoline, 4-[3-(2-pyridinyl)-1H-pyrazol-4-yl]); K02288 (3-[(6-amino-5-(3,4,5-trimethoxyphenyl)-3-pyridinyl]phenol]; and LDN-212854 (quinoline, 5-[6-[4-(1-piperazinyl)phenyl]pyrazolo[1,5-a]pyrimidin-3-yl]).

26. The chemical cocktail composition of claim 21, wherein the cAMP agonist is at least one selected from the group consisting of forskolin, isoproterenol, NKH 477 (a novel forskolin derivative), PACAP 1-27 (pituitary adenylate cyclase activating polypeptide receptor antagonist; PACAP antagonist), and PACAP 1-38 (PACAP antagonist).

27. The chemical cocktail composition of claim 21, wherein the histone demethylase inhibitor is at least one selected from the group consisting of parnate (tranylcypromine), SP2509, Ciclipirox, Daminozide, GSK J1, GSK J2, GSK J4, GSK J5, GSK LSD1, (R)-2-hydroxyglutaric acid, IOX1, JIB04, NSC636819, OG-L002, PBIT, RN 1 dihydrochloride, S2101, and TC-E 5002.

28. The chemical cocktail composition of claim 22, wherein the RAR agonist is at least one selected from the group consisting of TTNPB (4-[(E)-2-(5,6,7,8-tetrahydro-5,5,8,8-tetramethyl-2-naphthalenyl)-1-propenyl]benzoic acid, and arotinoid acid.

29. A method for preventing and treating neurological disorder, the method comprising a step of administering, to a subject having the neurological disorder, the glia-like cells that are differentiated from somatic cells according to any one of claims 1 to 12.
